# EUROPEAN PATENT APPLICATION

(11) **EP 4 613 876 A1**
(43) Date of publication of application: **10.09.2025**
(21) Application number: 24162466.7
(22) Date of filing: 08.03.2024
(51) Int. Cl.: C12Q 1/6858

(54) **NUCLEIC ACID QUANTIFICATION USING UNIVERSAL DETECTION PROBES**

(71) Applicant: Pxlence BV, 9200 Dendermonde (BE)
(72) Inventor: Vandesompele, Joke, 9200 Dendermonde (BE); Wils, Gertjan, 9200 Dendermonde (BE)
(74) Representative: Brantsandpatents bv

(57) **Abstract**

The current invention relates to a PCR detection system comprising a reaction mixture for analyzing, quantitating or detecting a target nucleic acid, wherein the reaction mixture comprises:
a. a first primer, wherein said first primer comprises a region configured to selectively hybridize to said target nucleic acid under amplification conditions and a first 5'-universal tail which is not complementary to the target nucleic acid sequence;
b. a first universal detection probe, wherein said first universal detection probe comprises a first fluorophore,
characterized in that the first 5'-universal tail of said first primer comprises a nucleotide sequence, the complement of which allows selective hybridization to a nucleotide sequence comprised in said first universal detection probe. The current invention further relates to methods and uses of aforementioned PCR detection system.

## Description

### FIELD OF THE INVENTION

The present invention relates to a PCR detection system comprising a universal detection probe. In further aspects, the present invention also relates to methods and uses for analyzing, quantitating or detecting a target nucleic acid using such a universal detection probe. The invention further relates to a universal detection probe having a loop with a specific nucleotide sequence.

### BACKGROUND

In PCR, the most commonly used methods for detection and quantitation of PCR amplification products are through the use of intercalating dyes, or through the use of target-specific oligonucleotide probes dual-labeled with fluorophores and quenchers. Such dual-labeled probes are generally favored over intercalating dyes because multiple different spectrally distinct fluorophores can be used on different probes to allows multiplexed detection and quantitation of multiple distinct DNA target species in a single reaction. However, the disadvantage of such probes is that they must be individually designed and tested for different target sequences, which increases both the time and costs associated with the PCR reaction.

The present invention aims to resolve at least some of the problems and disadvantages mentioned above.

### SUMMARY OF THE INVENTION

The present invention and embodiments thereof serve to provide a solution to one or more of above-mentioned disadvantages. To this end, the present invention relates to a PCR detection system according to claim 1. More specifically, the invention relates to a PCR detection system comprising a reaction mixture for analyzing, quantitating or detecting a target nucleic acid, wherein the reaction mixture comprises:
a. a first primer, wherein said first primer comprises a region configured to selectively hybridize to said target nucleic acid under amplification conditions and a first 5'-universal tail which is not complementary to the target nucleic acid sequence;
b. a first universal detection probe, wherein said first universal detection probe comprises a first fluorophore,
characterized in that the first 5'-universal tail of said first primer comprises a nucleotide sequence, the complement of which allows selective hybridization to a nucleotide sequence comprised in said first universal detection probe.

By providing a PCR detection system having a universal probe, the invention saves cost and time, as no target-specific probe must be designed and validated. Also, no longer unused stock remains, as a probe can be used to quantify any current and future targets. Furthermore, as no target-specific sequence is needed, very short amplicons can be designed.

Preferred embodiments of the PCR detection system are shown in any of the claims 2 to 8.

In a second aspect, the present invention relates to a method according to claim 9. More particular, the method as described herein relates to a PCR method for quantifying a target nucleic acid molecule by means of aforementioned PCR detection system, the method comprising: providing a reaction mixture comprising one or more target nucleic acid molecules and a primer, wherein said primer comprises a region configured to selectively hybridize to said target nucleic acid under amplification conditions and a 5'-universal tail which is not complementary to the target nucleic acid sequence, a universal detection probe, wherein said universal detection probe comprises a fluorophore, wherein the 5'-universal tail of said primer comprises a nucleotide sequence, the complement of which allows selective hybridization to a nucleotide sequence comprised in said universal detection probe; amplifying the target nucleic acid; measuring the fluorophore and thereby quantifying the target nucleic acid molecule.

The invention further relates to a PCR method for determining the genotype of a target nucleic acid molecule by means of aforementioned PCR detection system.

In a third aspect the present invention relates to a universal detection probe according to claim 12.

In a last aspect, the invention relates to use of aforementioned universal detection probe.

### DESCRIPTION OF FIGURES

The following description of the figures of specific embodiments of the invention is merely exemplary in nature and is not intended to limit the present teachings, their application or uses. Throughout the drawings, corresponding reference numerals indicate like or corresponding parts and features.
**Figure 1** shows a schematic representation of a forward allele-specific primer comprising a 5'-universal tail of a PCR detection system according to an embodiment of the present invention.
**Figure 2** shows a schematic representation of three possible configurations (hairpin (left), horseshoe (middle), shell (right)) of molecular beacon probes according to an embodiment of the present invention.
**Figure 3** Fourteen hairpin-style probes were tested, consisting of a 13-mer loop sequence and a 5-mer stem sequence. The 13-mer loop sequences (A-N) were designed and empirically evaluated. The loop sequence is the reverse complement sequence of a 5' tail modified primer so amplicons are detected.
**Figure 4** Melting curves of PCR amplicons are shown in Figures 4 A-C with three different probes (1, 2 and 3, respectively Figure 4A, 4B and 4C) and four different loop lengths (13 nucleotides (circle), 18 nucleotides (triangle), 21 nucleotides (square) and 24 nucleotides (diamond)) for a MYCN target gene assay using DNA as input (highest 4 lines) and NTC (lowest 4 lines).
**Figure 5** Results for three different stem lengths (5, 6, and 7 nucleotides) and sequences using loop A are shown for a MYCN (Figure 5 A, C, E respectively) and BIRC5 assay (Figure 5 B, D, F respectively). Annealing temperature was tested at 50, 55 or 60 °C (left, middle and right panel).
**Figure 6** The same primer (for the wild-type BRAF gene) was modified at its 5' end to be detected by 6 different probes (different loop sequence and different fluorophore) according to an embodiment of the current invention in separate digital PCR reactions using human gDNA as input.
**Figure 7A** shows the results of two shell probes (Shell_B (diamond) and shell Y (circles)) according to an embodiment of the invention to detect human genomic DNA on a CFX384 qPCR system. Shell probes work better in qPCR than stem-loop hairpin probes (higher RFU).
**Figure 7B** shows results for three different probes according to an embodiment of the invention: Shell_B (A01 to C01), hairpin_B (A02 to C02) and shell_Y (E01 to G01) on human gDNA with their respective no-template-controls (D01, D02 and H01).
**Figure 8** shows test results for 4 different probes according to an embodiment of the invention (hairpin, shell, horseshoe, horseshoe).
**Figure 9** shows seven strategies to detect a single target with two different probes in two different detection channels.
Figures 10A-B show 1D plots for all 7 strategies as described in example 2 and figure 9 with probes PXL-RBO-001-P-FAM (A) and PXL-RBO-001-B-HEX (B) for target BRAFV600E_WT.
**Figure 11** shows a 2D scatterplot of strategy 7 with PXL-RBO-001-P-FAM (y-axis) and PXL-RBO-001-B-HEX (x-axis), with 1x probe concentration (125 nM, cloud 3) and 2x probe concentration (250 nM, cloud 2). For visualization purposes, the same target was also detected in separate reactions using single probe (PXL-RBO-001-P-FAM (cloud 1) and PXL-RBO-001-B-HEX (cloud 4).
**Figure 12** shows optimization of primer and probe concentrations of a PCR detection system according to an embodiment of the current invention as described in example 3.
**Figure 13** shows three assays with either slow or fast cooling (TOMM7, NOP10 and BRAF-WT) with their respective probes (PXL-RBO-001-P-FAM or PXL-RBO-001-B-HEX) according to an embodiment of the invention. Slow cooling was 0.5 °C/s and fast cooling 4 °C/s. Figure 13A shows TOMM7 and PXL-RBO-001-P-FAM with slow cooling (0.5 °C/s). Figure 13B shows TOMM7 and PXL-RBO-001-P-FAM with fast cooling (4 °C/s). Figure 13C shows NOP10 and BRAF-WT with probe PXL-RBO-001-B-HEX with slow cooling (0.5 °C/s). Figure 13D shows NOP10 and BRAF-WT with probe PXL-RBO-001-B-HEX with fast cooling (4 °C/s).
**Figure 14** illustrates that the probes of the current invention work on different instruments for both qPCR and dPCR (A: QX600 (Bio-Rad), B: QIAcuity (Qiagen), C: Nio+ system (Stilla Technologies)).
**Figure 15** shows a PCR assay to detect the BRAF V600E mutation according to an embodiment of the invention having an allele-specific primer that is modified for detection with a non-target specific probe through 5' end modification of the primer.

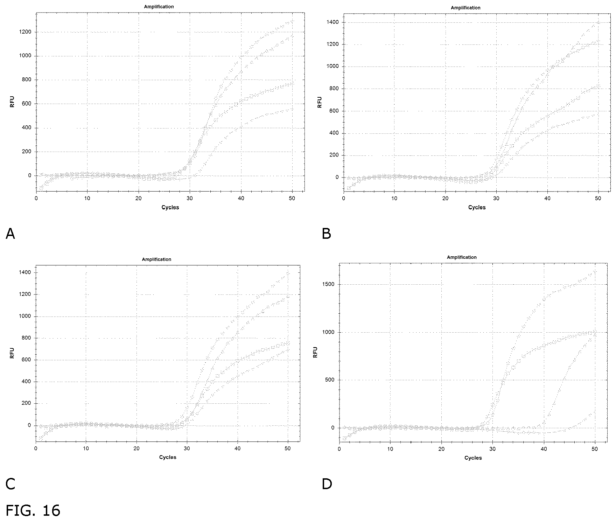

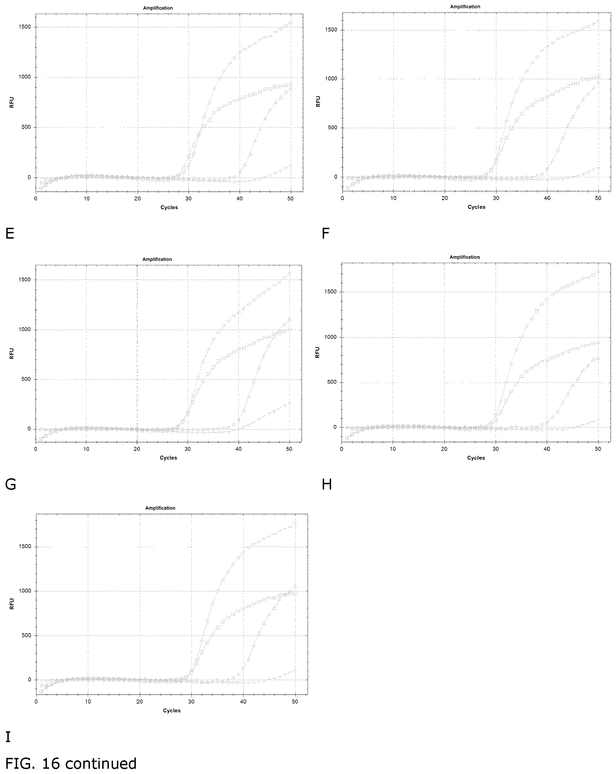

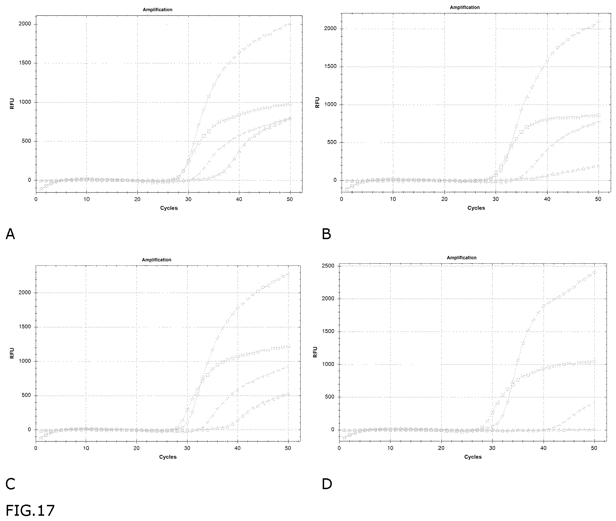

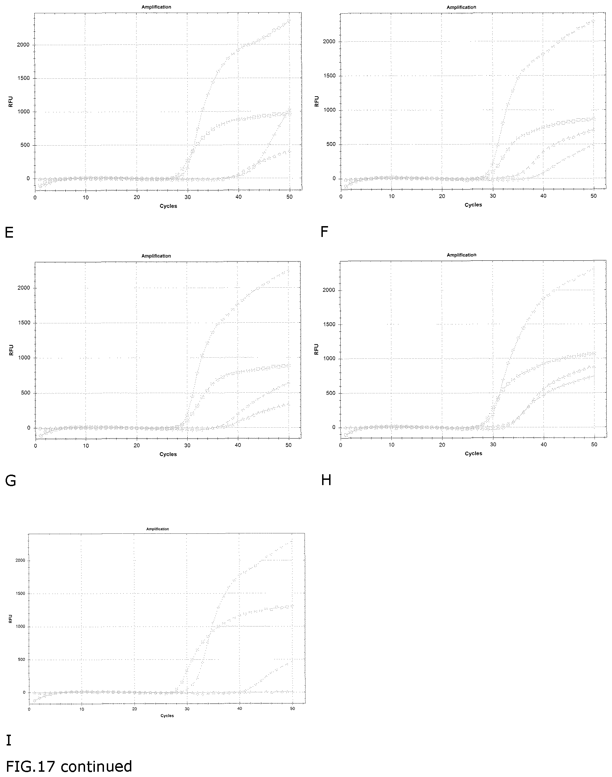

**Figure 16** and **Figure 17** In a duplex assay according to an embodiment of the current invention, WT (Wild Type) and MUT (mutant) primer differ with their 5' end probe modification and mismatch position or sequence. This results into nine different mismatch position based combinations. These 9 position possibilities were tested in singleplex using qPCR with either WT assay and WT input (square), WT assay and MUT input (diamond), MUT assay and WT input (triangle) and MUT assay and MUT input (circle). Assays were designed for a mutation in the AXIN1 (Figure 16) or PTPRD gene (Figure 17). Amplification plots are shown for each mismatch combination as described in Table 6.
**Figure 18** show the results of three cycle protocols as described in Table 7. Cycle protocol 1 is depicted in Figure 18A, cycle protocol 2 is depicted in Figure 18B, cycle protocol 1 is depicted in Figure 18C.

### DETAILED DESCRIPTION OF THE INVENTION

The present invention relates to a PCR detection system comprising a universal detection probe. The present invention also relates to methods and uses for analyzing, quantitating or detecting a target nucleic acid using such a universal detection probe. The present invention also relates to an optimized universal detection probe.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

As used herein, the terms "amplification", "nucleic acid amplification", or "amplifying" refer to the production of multiple copies of a nucleic acid template, or the production of multiple nucleic acid sequence copies that are complementary to the nucleic acid template. The terms (including the term "polymerizing") may also refer to extending a nucleic acid template (e.g., by polymerization). The amplification reaction may be a polymerase-mediated extension reaction such as, for example, a polymerase chain reaction (PCR). However, any of the known amplification reactions may be suitable for use as described herein. The term "amplifying" that typically refers to an "exponential" increase in target nucleic acid may be used herein to describe both linear and exponential increases in the numbers of a select target sequence of nucleic acid.

As used herein, the term "under amplification conditions" refers to conditions that that enable amplification.

The term "reaction mixture" may refer to an aqueous solution including the various (some or all) reagents used to amplify a target nucleic acid. Such reactions may also be performed using solid supports (e.g., an array). The reactions may also be performed in single or multiplex format as desired by the user. These reactions typically include enzymes, aqueous buffers, salts, amplification primers, target nucleic acid, and nucleoside triphosphates. Depending upon the context, the mixture may be either a complete or incomplete amplification reaction mixture. The method used to amplify the target nucleic acid may be any available to one of skill in the art. Any in vitro means for multiplying the copies of a target sequence of nucleic acid may be utilized. These include linear, logarithmic, and/or any other amplification method. While this disclosure may generally discuss PCR as the nucleic acid amplification reaction, it is expected that the oligonucleotides, reaction mixtures and/or compositions described herein should be effective in other types of nucleic acid amplification reactions, including polymerase-mediated amplification reactions (such as helicase-dependent amplification (HDA), recombinasepolymerase amplification (RPA), and rolling circle amplification (RCA)), as well as ligase-mediated amplification reactions (such as ligase detection reaction (LDR), ligase chain reaction (LCR), and gap-versions of each), and combinations of nucleic acid amplification reactions such as LDR and PCR. For example, the oligonucleotides, reaction mixtures and/or compositions disclosed herein may be used in, for example, various ligation-mediated reactions, where for example ligation probes are employed as opposed to PCR primers. Additional exemplary methods include polymerase chain reaction (PCR), isothermal procedures (using one or more RNA polymerases), strand displacement, partial destruction of primer molecules, ligase chain reaction (LCR), QJ RNA replicase systems, RNA transcription-based systems (e.g., TAS, 3SR), rolling circle amplification (RCA), and strand displacement amplification (SDA), among others. These systems, along with the many other systems available to the skilled artisan, may be suitable for use in polymerizing and/or amplifying target nucleic acids for use as described herein.

In certain embodiments, amplification techniques comprise at least one cycle of amplification, for example, but not limited to, the steps of: denaturing a double-stranded nucleic acid to separate the component strands; hybridizing a primer to a target flanking sequence or a primer-binding site of an amplicon (or complements of either, as appropriate); and synthesizing a strand of nucleotides in a template-dependent manner using a DNA polymerase. The cycle may or may not be repeated. In certain embodiments, a cycle of amplification comprises a multiplicity of amplification cycles, for example, but not limited to 20 cycles, 25 cycles, 30 cycles, 35 cycles, 40 cycles, 45 cycles or more than 45 cycles of amplification.

In some embodiments, amplifying comprises thermocycling using an instrument. In certain embodiments, single- stranded amplicons are generated in an amplification reaction, for example, but not limited to asymmetric PCR or A-PCR, or asynchronous thermal cycling.

In some embodiments, amplification comprises a two-step reaction including without limitation, a pre-amplification step wherein a limited number of cycles of amplification occur (for example, but not limited to, 2, 3, 4, or 5 cycles of amplification), then the resulting amplicon is generally diluted and portions of the diluted amplicon are subjected to additional cycles of amplification in a subsequent amplification step.

The terms "amplicon" and "amplification product" as used herein generally refer to the product of an amplification reaction. An amplicon may be double-stranded or single- stranded, and may include the separated component strands obtained by denaturing a double-stranded amplification product. In certain embodiments, the amplicon of one amplification cycle can serve as a template in a subsequent amplification cycle.

The terms "denaturing" and "denaturation" as used herein refer to any process in which a double-stranded polynucleotide, including without limitation, a genomic DNA (gDNA) fragment comprising at least one target nucleic acid, a double-stranded amplicon, or a polynucleotide comprising at least one double-stranded segment is converted to two single-stranded polynucleotides or to a single-stranded or substantially single- stranded polynucleotide, as appropriate. Denaturing a double-stranded polynucleotide includes, without limitation, a variety of thermal and chemical techniques which render a double-stranded nucleic acid single-stranded or substantially single- stranded, for example but not limited to, releasing the two individual single-stranded components of a double-stranded polynucleotide or a duplex comprising two oligonucleotides. Those in the art will appreciate that the denaturing technique employed is generally not limiting unless it substantially interferes with a subsequent annealing or enzymatic step of an amplification reaction, or in certain methods, the detection of a fluorescent signal.

The terms "complementarity" and "complementary" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% complementarity refers to the situation in which each nucleotide unit of one polynucleotide strand or region can hydrogen bond with each nucleotide unit of a second polynucleotide strand or region.

The "complement" or "complement sequence" are interchangeable and refer to the complementary sequence of a certain sequence.

The terms "annealing" and "hybridizing", including, without limitation, variations of the root words "hybridize" and "anneal", are used interchangeably and mean the nucleotide base-pairing interaction of one nucleic acid with another nucleic acid that results in the formation of a duplex, triplex, or other higher-ordered structure. The primary interaction is typically nucleotide base specific, e.g., A:T, A:U, and G:C, by Watson-Crick and Hoogsteen-type hydrogen bonding. In certain embodiments, base-stacking and hydrophobic interactions may also contribute to duplex stability. Conditions under which primers and probes anneal to complementary sequences are well known in the art.

In general, whether such annealing takes place is influenced by, among other things, the length of the complementary portions of the primers and their corresponding binding sites in the target flanking sequences and/or amplicons, or the corresponding complementary portions of a reporter probe or a detection probe and its binding site; the pH; the temperature; the presence of mono- and divalent cations; the proportion of G and C nucleotides in the hybridizing region; the viscosity of the medium; and the presence of denaturants. Such variables influence the time required for hybridization. Thus, the preferred annealing conditions will depend upon the particular application. Such conditions, however, can be routinely determined by persons of ordinary skill in the art, without undue experimentation. Preferably, annealing conditions are selected to allow the primers and/or probes to selectively hybridize with a complementary sequence in the corresponding target flanking sequence or amplicon, but not hybridize to any significant degree to different target nucleic acids or non-target sequences in the reaction composition at the second reaction temperature.

The term "selectively hybridize" and variations thereof, means that, under appropriate stringency conditions, a given sequence (for example, but not limited to, a primer or probe) anneals with a second sequence comprising a complementary string of nucleotides (for example, but not limited to, a target flanking sequence or primer binding site of an amplicon), but does not anneal to undesired sequences, such as non-target nucleic acids, probes, or other primers. Typically, as the reaction temperature increases toward the melting temperature of a particular double-stranded sequence, the relative amount of selective hybridization generally increases and mis-priming generally decreases. In this specification, a statement that one sequence hybridizes or selectively hybridizes with another sequence encompasses situations where the entirety of both of the sequences hybridize or selectively hybridize to one another, and situations where only a portion of one or both of the sequences hybridizes or selectively hybridizes to the entire other sequence or to a portion of the other sequence.

As used herein, the term "genomic DNA" or "gDNA" refers to the total DNA from an organism, that is, the whole complement of an organism's DNA. Typically, this includes both the intron and exon sequences and the non-coding regulatory sequences, such as the promoter and enhancer sequences.

As used herein, the term "complementary DNA" or "cDNA" or "copy DNA" refers to a type of DNA molecule that is synthesized from a messenger RNA (mRNA) template through a process called reverse transcription. The enzyme reverse transcriptase is used to catalyze the synthesis of cDNA, which is complementary to the mRNA template.

The terms "nucleic acid polymerase" and "DNA polymerase" are used herein in a broad sense and refers to any polypeptide that can catalyze the 5'-to-3' extension of a hybridized primer by the addition of deoxyribonucleotides and/or certain nucleotide analogs in a template-dependent manner. For example, but not limited to, the sequential addition of deoxyribonucleotides to the 3 '-end of a primer that is annealed to a nucleic acid template during a primer extension reaction. Non-limiting examples of DNA polymerases include RNA-dependent DNA polymerases, including without limitation, reverse transcriptases, and DNA-dependent DNA polymerases. It is to be appreciated that certain DNA polymerases (for example, but not limited to certain eubacterial Type A DNA polymerases and Taq DNA polymerase) may further comprise a structure-specific nuclease activity and that when an amplification reaction comprises an invasive cleavage reaction.

As used herein, the term "Tm" is used in reference to melting temperature. The melting temperature is the temperature at which a population of double-stranded nucleic acid molecules becomes half dissociated into single strands.

The term "minor groove binder" as used herein refers to a small molecule that fits into the minor groove of double-stranded DNA, sometimes in a sequence specific manner. Generally, minor groove binders are long, flat molecules that can adopt a crescent-like shape and thus, fit snugly into the minor groove of a double helix, often displacing water. Minor groove binding molecules typically comprise several aromatic rings connected by bonds with torsional freedom, for example, but not limited to, furan, benzene, or pyrrole rings.

As used herein the terms "Ct " and "cycle threshold" refer to the time at which fluorescence intensity is greater than background fluorescence. They are characterized by the point in time (or PCR cycle) where the target amplification is first detected. Consequently, the greater the quantity of target DNA in the starting material, the faster a significant increase in fluorescent signal will appear, yielding a lower Ct .

As used herein, the term "Cq" and "quantification cycle" refer to the fractional number of cycles that were needed for the fluorescence to reach a quantification threshold.

As used herein, the term "probe" refers to synthetic or biologically produced nucleic acids (DNA or RNA) which, by design or selection, contain specific nucleotide sequences that allow them to hybridize, under defined stringencies, specifically (i.e., preferentially) to target nucleic acid sequences. In the present teachings, probes may be labeled, e.g., with a fluorescent dye, or a pair of labels comprising a fluorophore reporter dye and a quencher to enable detection.

As used herein, the term "template" is interchangeable with "target molecule" or "target nucleic acid" and refers to a double-stranded or single-stranded nucleic acid molecule which is to be amplified, copied or extended, synthesized, or sequenced. In the case of a double-stranded DNA molecule, denaturation of its strands to form a first and a second strand is performed to amplify, sequence, or synthesize these molecules. A primer, complementary to a portion of a template is hybridized under appropriate conditions and the polymerase (DNA polymerase or reverse transcriptase) may then synthesize a nucleic acid molecule complementary to said template or a portion thereof. The newly synthesized molecule, according to the present disclosure, may be equal or shorter in length than the original template. Mismatch incorporation during the synthesis or extension of the newly synthesized molecule may result in one or a number of mismatched base pairs. Thus, the synthesized molecule need not be exactly complementary to the template. The template may be an RNA molecule, a DNA molecule, or a DNA/RNA hybrid molecule. A newly synthesized molecule may serve as a template for subsequent nucleic acid synthesis or amplification.

The terms "nucleic acid binding dye" or "double-stranded nucleic acid intercalating dye" as used herein refer to a fluorescent molecule that is specific for a double-stranded polynucleotide or that at least shows a substantially greater fluorescent enhancement when associated with double-stranded polynucleotides than with a single stranded polynucleotide. Typically, nucleic acid binding dye molecules associate with double-stranded segments of polynucleotides by intercalating between the base pairs of the double-stranded segment, but binding in the major or minor grooves of the double- stranded segment, or both. Non-limiting examples of nucleic acid binding dyes include ethidium bromide, DAPI, Hoechst derivatives including without limitation Hoechst 33258 and Hoechst 33342, intercalators comprising a lanthanide chelate (for example, but not limited to, a naphthalene diimide derivative carrying two fluorescent tetradentate-diketone-Eu3+ chelates (NDI-(BHHCT-Eu3+ )2 ), and certain unsymmetrical cyanine dyes such as SYBR^{®} Green, SYBR^{®} GreenER^{™} and PicoGreen^{®} and their derivatives.

As used herein, the terms "polynucleotide", "oligonucleotide," and "nucleic acid" are used interchangeably and refer to single- stranded and double-stranded polymers of nucleotide monomers, including without limitation, 2'-deoxyribonucleotides (DNA) and ribonucleotides (RNA) linked by internucleotide phosphodiester bond linkages, or internucleotide analogs, and associated counter ions, e.g., H⁺, NH₄⁺, trialkylammonium, Mg²⁺, Na⁺, and the like. A polynucleotide may be composed entirely of deoxyribonucleotides, entirely of ribonucleotides, or chimeric mixtures thereof and may include nucleotide analogs. The nucleotide monomer units may comprise any of the nucleotides described herein, including, but not limited to, nucleotides and/or nucleotide analogs. Polynucleotides typically range in size from a few monomeric units, e.g., 5-40 when they are sometimes referred to in the art as oligonucleotides, to several thousands of monomeric nucleotide units. Unless denoted otherwise, whenever a polynucleotide sequence is represented, it will be understood that the nucleotides are in the 5'-to-3' order from left to right and that "A" denotes deoxyadenosine, "C" denotes deoxycytosine, "G" denotes deoxyguanosine, "T" denotes deoxythymidine, and "U" denotes deoxyuridine, unless otherwise noted.

The term "nucleotide" refers to a phosphate ester of a nucleoside, e.g., triphosphate esters, wherein the most common site of esterification is the hydroxyl group attached at the C-5 position of the pentose.

The term "nucleoside" refers to a compound consisting of a purine, deazapurine, or pyrimidine nucleoside base, e.g., adenine, guanine, cytosine, uracil, thymine, deazaadenine, deazaguanosine, and the like, linked to a pentose at the 1' position, including 2'-deoxy and 2'- hydroxyl forms. When the nucleoside base is purine or 7-deazapurine, the pentose is attached to the nucleobase at the 9- position of the purine or deazapurine, and when the nucleobase is pyrimidine, the pentose is attached to the nucleobase at the 1 -position of the pyrimidine.

The term "analog" includes synthetic analogs having modified base moieties, modified sugar moieties, and/or modified phosphate ester moieties. Phosphate analogs generally comprise analogs of phosphate wherein the phosphorous atom is in the +5 oxidation state and one or more of the oxygen atoms is replaced with a non-oxygen moiety, e.g. sulfur. Exemplary phosphate analogs include: phosphorothioate, phosphorodithioate, phosphoroselenoate, phosphorodiselenoate, phosphoroanilothioate, phosphoranilidate, phosphoramidate, boronophosphates, including associated counterions, e.g., H⁺, NH₄⁺, Na⁺. Exemplary base analogs include: 2,6-diaminopurine, hypoxanthine, pseudouridine, C-5-propyne, isocytosine, isoguanine, 2-thiopyrimidine. Exemplary sugar analogs include: 2'- or 3'-modifications where the 2'- or 3'-position is hydrogen, hydroxy, alkoxy, e.g., methoxy, ethoxy, allyloxy, isopropoxy, butoxy, isobutoxy and phenoxy, azido, amino or alkylamino, fluoro, chloro, and bromo.

### Detailed description

In PCR, the most commonly used methods for detection and quantitation of PCR amplification products are through the use of intercalating dyes, or through the use of target-specific oligonucleotide probes dual-labeled with fluorophores and quenchers. Such dual-labeled probes are generally favored over intercalating dyes because multiple different spectrally distinct fluorophores can be used on different probes to allows multiplexed detection and quantitation of multiple distinct DNA target species in a single reaction. However, the disadvantage of such probes is that they must be individually designed and tested for different target sequences, which increases both the time and costs associated with the PCR reaction.

As such, in a first aspect, the invention relates to a PCR detection system comprising a reaction mixture for analyzing, quantitating or detecting a target nucleic acid, wherein the reaction mixture comprises:
a. a first primer, wherein said first primer comprises a region configured to selectively hybridize to said target nucleic acid under amplification conditions and a first 5'-universal tail which is not complementary to the target nucleic acid sequence;
b. a first universal detection probe, wherein said first universal detection probe comprises a first fluorophore,
characterized in that the first 5'-universal tail of said first primer comprises a nucleotide sequence, the complement of which allows selective hybridization to a nucleotide sequence comprised in said first universal detection probe.

Likewise, in an further aspect, the invention relates to a PCR method for quantifying a target nucleic acid molecule by means of aforementioned PCR detection system, the method comprising: providing a reaction mixture comprising one or more target nucleic acid molecules and a primer, wherein said primer comprises a region configured to hybridize to said target nucleic acid under amplification conditions and a 5'-universal tail which is not complementary to the target nucleic acid sequence, a universal detection probe, wherein said universal detection probe comprises a fluorophore, wherein the 5'-universal tail of said primer comprises a nucleotide sequence, the complement of which allows hybridization to a nucleotide sequence comprised in said universal detection probe; amplifying the target nucleic acid; measuring the fluorophore and thereby quantifying the target nucleic acid molecule.

By providing a PCR detection system and a method having a universal probe, the invention enables a faster turnaround time when designing and developing assays as compared to probes known from the state of the art. The use of universal probes saves cost and time, as no target-specific probe must be designed and validated. Another advantage is that there is no longer unused stock as a probe can be used to quantify any current and future targets.

Furthermore, as no target-specific sequence is needed, very short amplicons can be designed, even without template sequence other than the primers (for instance when using primers that are head-to-head or overlap 1 or up to 5 nucleotides). In an embodiment, wherein a forward and reverse primer overlap by their 3' ultimate nucleotide, an amplicon can be produced having an amplicon size of the forward primer plus the reverse primer minus 1 nucleotide. Likewise, when a forward and reverse primer overlap by their 3' two final nucleotides, an amplicon can be produced having an amplicon size of the forward primer plus the reverse primer minus two nucleotides. When a forward and reverse primer overlap by their 3'three final nucleotides, an amplicon can be produced having an amplicon size of the forward primer plus the reverse primer minus three nucleotides. When a forward and reverse primer overlap by their 3' four final nucleotides, an amplicon can be produced having an amplicon size of the forward primer plus the reverse primer minus four nucleotides. And when a forward and reverse primer overlap by their 3' five final nucleotides, an amplicon can be produced having an amplicon size of the forward primer plus the reverse primer minus five nucleotides.

The invention is suitable for any PCR quantification in which the abundance of target if measured (e.g. gene expression, gene copy number, pathogens, vectors, ... for any species or synthetic sequence).

As discussed further below, the current invention further allows to do multiplex detection and quantification of different targets, by modifying one primer from different primer pairs (targets) with a different tail sequence.

In an embodiment, the primer pair (forward plus reverse primer) consists of one primer having a 5'-universal tail (further referred to as "modified primer") and one "unmodified primer" (or "regular primer")."Unmodified primer" as used herein refers to a primer not having such a 5'-universal tail.

In an embodiment, the PCR detection system further includes a drop-off hydrolysis probe for mutation screening. In such an embodiment, the modified primer detects the target DNA molecule (irrespective of it being wild-type or mutant), and the drop-off hydrolysis probe detects any mutation underlying the probe.

Drop-off assays known from the prior art normally use a target specific wild-type probe adjacent to a target-specific drop-off probe. The first probe (Probe 1) covers a non-variable sequence, adjacent to the target region and the second probe (Probe 2) is complementary to the WT sequence of the target region where mutations are expected. While the first probe quantifies the total number of amplifiable molecules in the reaction, the second probe discriminates WT and MUT alleles by sub-optimal hybridization to mutant sequences. Probe 2 can identify multiple types of mutations in the target region (single or multiple nucleotide substitutions, deletion, etc.). The number of amplifiable molecules in the reaction is quantified by the modified primer and the type and amount of mutations with a hydrolysis probe.

By omitting the wild-type probe, the current invention allows to generate much smaller amplicons (important for fragmented/degraded DNA) and requires only one target specific probe.

In an alternative embodiment, both the forward and the reverse primer are modified with a 5'-universal tail.

For instance, the invention allows to detect a fusion transcript by combining a forward primer having a first 5'-universal tail directed against the 5' fusion partner gene (to be recognized by a first universal detection probe comprising fluorophore A), and a reverse primer having a second 5'-universal tail directed against the 3' fusion partner gene (to be recognized by a second universal detection probe comprising fluorophore B). When there is a fusion gene, one forward and one reverse primer will form an amplicon that fluoresces in both A and B channel.

Such a PCR detection system can for instance also be used for cancer fusion transcript detection when the breakpoint location is not known; in each exon of the 5' fusion partner gene, a forward primer having a first 5'-universal tail is designed (to be recognized by a first universal detection probe comprising fluorophore A), in each exon of the 3' fusion partner gene, a reverse primer having a second 5'-universal tail is designed (to be recognized by a second universal detection probe comprising fluorophore B); when there is a fusion gene, one forward and one reverse primer will form an amplicon that fluoresces in both the A and B channel.

The target nucleic acid may be obtained from any source, and may comprise any number of different compositional components. For example, the target may be a nucleic acid (e.g., DNA or RNA), cDNA, transfer RNA (tRNA), small interfering RNA (siRNA), microRNA (miRNA), or other mature small RNA, and may comprise nucleic acid analogs or other nucleic acid mimics. The target may be methylated, non-methylated, or both. The target may be bisulfite-treated and non-methylated cytosines converted to uracil. Further, it will be appreciated that "target nucleic acid" may refer to the target nucleic acid itself, as well as surrogates thereof, for example, amplification products and native sequences. The target molecules of the present teachings may be derived from any number of sources, including without limitation, viruses, archaea, protists, prokaryotes and eukaryotes, for example, but not limited to, plants, fungi, and animals. These sources may include, but are not limited to, whole blood, a tissue biopsy, lymph, bone marrow, amniotic fluid, hair, skin, semen, biowarfare agents, anal secretions, vaginal secretions, perspiration, saliva, buccal swabs, various environmental samples (for example, agricultural, water, and soil), research samples generally, purified samples generally, cultured cells and lysed cells. It will be appreciated that target nucleic acids may be isolated from samples using any of a variety of procedures known in the art. It will be appreciated that target nucleic acids may be cut or sheared prior to analysis, including the use of such procedures as mechanical force, sonication, restriction endonuclease cleavage, or any method known in the art. In general, the target nucleic acids of the present teachings will be single-stranded, though in some embodiments the target nucleic acids may be double-stranded, and a single-strand may result from denaturation.

Nucleic acid amplification often is based on nucleic acid synthesis by a nucleic acid polymerase or reverse transcriptase. Many such nucleic acid polymerases or reverse transcriptases require the presence of a primer that may be extended to initiate such nucleic acid synthesis.

As used herein, the term "primer" refers to a synthetically or biologically produced single-stranded oligonucleotide that is extended by covalent bonding of nucleotide monomers during amplification or polymerization of a nucleic acid molecule.

The primers of the current invention comprise a region configured to selectively hybridize to said target nucleic acid under amplification conditions and a first 5'-universal tail which is not complementary to the target nucleic acid sequence, but comprises a nucleotide sequence, the complement of which allows selective hybridization to a nucleotide sequence comprised in said first universal detection probe.

The primers of the current invention can comprise natural and/or modified nucleotide residues and internucleotide linkages. Alternative nucleic acid backbones can include for instance but are not limited to phosphorothioate, phosphoroselenoate, alkyl phosphotriester, aryl phosphotriester, alkyl phosphonate, aryl phosphonate, phosphoboronate, morpholino nucleic acid (MNA), locked nucleic acids (LNA), peptide nucleic acids (PNA). In an embodiment, the primer comprises a blocking agent at its 5'end in order to block the 5'end. In an embodiment, said blocking agent is a hydrophobic 6-carbon spacer.

In certain embodiments, the 5'-universal tail of the primer has a length of about 8 nucleotides to about 30 nucleotides. Preferably, the 5'-universal tail of the primer has a length of about 15 nucleotides to about 20 nucleotides. Most preferably, the 5'-universal tail of the primer has a length of about 18 nucleotides.

In an embodiment, said 5'-universal tail comprises a nucleotide sequence having a sequence identity of at least 90% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 91% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 92% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 93% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 94% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 95% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 96% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 97% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 98% to a nucleotide sequence comprised in said universal detection probe, wherein said nucleotide sequence showing sequence identity comprises at least 8 nucleotides. In an embodiment, said 5'-universal tail comprises a nucleotide sequence having a sequence identity of at least 99% to a nucleotide sequence comprised in said universal detection probe, wherein said nucleotide sequence showing sequence identity comprises at least 8 nucleotides.

In an embodiment, said nucleotide sequence showing sequence identity consists of 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides or 30 nucleotides. In a preferred embodiment, said nucleotide sequence showing sequence identity consists of between 15 and 20 nucleotides, such as 18 nucleotides. When modified nucleotides are used (such as LNA modified nucleotides having a higher melting temperature Tₘ), said nucleotide sequence showing sequence identity can consist of fewer nucleotides.

The terms "percent sequence identity" or "percent identical" as used herein in reference to two or more nucleotide sequences is calculated by (i) comparing two optimally aligned sequences over a window of comparison (the "alignable" region or regions), (ii) determining the number of positions at which the identical nucleic acid base (for nucleotide sequences) occurs in both sequences to yield the number of matched positions, (iii) dividing the number of matched positions by the total number of positions in the window of comparison, and then (iv) multiplying this quotient by 100% to yield the percent identity. If the "percent identity" is being calculated in relation to a reference sequence without a particular comparison window being specified, then the percent identity is determined by dividing the number of matched positions over the region of alignment by the total length of the reference sequence.

Accordingly, for purposes of the present application, when two sequences (query and subject) are optimally aligned (with allowance for gaps in their alignment), the "percent identity" for the query sequence is equal to the number of identical positions between the two sequences divided by the total number of positions in the query sequence over its length (or a comparison window), which is then multiplied by 100%.

In a further embodiment, said 5'-universal tail of said primer comprises a nucleotide sequence which is a 100% identical to a nucleotide sequence comprised in said universal detection probe, wherein said identical nucleotide sequence comprises at least 8 nucleotides. Preferably said identical nucleotide sequence consist of more than 8 nucleotides, such as 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides. Preferably said identical nucleotide sequence consists of 18 nucleotides.

In an aspect, said primer having said 5'-universal tail is a forward primer. In an aspect, said primer having said 5'-universal tail is a reverse primer. As used herein, a "forward primer" hybridizes to the anti-sense strand of dsDNA, and a "reverse primer" hybridizes to the sense strand of dsDNA. In an aspect, the forward primer comprises DNA. In an aspect, the reverse primer comprises DNA. In an aspect, the forward primer comprises RNA. In an aspect, the reverse primer comprises RNA.

Preferably the unmodified primer has a higher starting PCR concentration than the primer comprising the 5'-universal tail sequence ('asymmetric' primer concentrations) in order to create single-stranded templates to which the detection probe can bind.

As such, in a preferred embodiment of the invention, the PCR method involves non-symmetric exponential amplification resulting in an excess of amplicons having the complement of the 5'-universal tail sequence incorporated into the 3' end. This is advantageous as the universal detection probe selectively hybridizes to said complement of the 5'-universal tail sequence.

As such, in a preferred embodiment, an asymmetric PCR is performed, wherein the concentration of the primer (be it forward or reverse) comprising the 5'-universal tail is lower than the unmodified primer of the primer pair.

For instance, when the forward primer comprises the 5'-universal tail sequence, the concentration of said forward primer is lower than the reverse primer of the primer pair. This will lead to non-symmetric exponential amplification, resulting in an excess of (-) amplicons. In a next step the detection probe will bind to the complement of the 5'-universal tail sequence incorporated in the 3' end of the excess (-) amplicons.

In an embodiment, the PCR reaction comprises 100 nM primer comprising the 5'-universal tail sequence and 300 nM unmodified primer.

In an embodiment, overlapping forward and reverse primer pairs are used (or + and - strand primer pairs) to avoid polymerase errors; with primers that overlap at their 3' end (at least 1, up to 5 nucleotides). By using such overlapping forward and reverse primer pairs one can avoid that the polymerase induces errors in the critical region to detect and quantify mutant alleles.

In the situation where the universal detection probe selectively hybridizes to a nucleotide sequence comprised in the complement sequence of the 5'-universal tail of said primer, the probe will provide a signal which can be detected and used for analyzing, quantitating or detecting the target nucleic acid.

As used herein, the term "detection probe" refers to a molecule used in an amplification reaction, typically for quantitative or real-time PCR analysis, as well as end-point analysis. The term "end-point" measurement refers to a method where data collection occurs only once the reaction has been stopped. One specific embodiment of end-point measurement is a digital PCR assay, including assays carried out in many different reaction chambers in a thermal cycler, and droplet digital PCR (ddPCR) assays carried out in many different droplets in a thermal cycler.

The terms "real-time" and "real-time continuous" are interchangeable and refer to a method where data collection occurs through periodic monitoring during the course of the polymerization reaction. Thus, the methods combine amplification and detection into a single step.

As used herein, the term "quantitative PCR" or "qPCR" refers to the use of the polymerase chain reaction (PCR) to quantify gene expression.

Such detection probes may be used to monitor the amplification of the target polynucleotide. In some embodiments, detection probes present in an amplification reaction are suitable for monitoring the amount of amplicon(s) produced as a function of time.

Additional exemplary methods for polymerizing and/or amplifying and detecting target nucleic acids suitable for use as described herein involve "molecular beacons".

The inventors performed experiments to optimize the universal detection probe, more specifically to optimize the loop length and nucleotide sequence thereof (see example 1). The inventors found that the nucleotide sequence of said loop should comprise:
- a GC content between 55 and 70%;
- a melting temperature between 60 °C and 70 °C;
and that said nucleotide sequence should not comprise:
- more than three consecutive identical nucleotides;
- a deoxycytosine (C) or deoxyguanosine (G) at the 5' end of the loop.

The melting temperature refers to PCR conditions having 50 mM "+ ions" (e.g. K⁺, Na⁺), 3 mM "++ ions" (e.g. Mg²⁺⁾, 1.2 mM dNTPs and 125 nM probe.

Furthermore, said nucleotide sequence of said loop should not form secondary structures and should not show complementarity to the human genome.

Nor should the probes show complementarity to itself. When multiple probes are used in one assay, the probes should not show complementarity to each other. Preferably the different probes have no more than six nucleotides in a row that are complementary.

In a preferred embodiment, the nucleotide sequence of the loop comprises more C than G nucleotides.

Furthermore, the nucleotide sequence of said loop should comprise a length of between 8 and 24 nucleotides. In an embodiment, the loop consists of 8, 9, 10, 11, 12, 13, 14, 15, 16, 17, 18, 19, 20, 21, 22, 23 or 24 nucleotides. In a preferred embodiment, the loop consists of 15-20 nucleotides, such as 18 nucleotides.

The inventors discovered that the longer the loop, the greater the difference from the gDNA, resulting in a better signal-to-noise (see example 1). A loop length of 18 nucleotides was selected as optimal as longer would also increase the length of the modified primer (shorter primers are preferred to minimize complexity and cross-reactivity).

As such, in a further aspect, the invention relates to a universal detection probe. At least part of said universal detection probe forms a loop, characterized in that the nucleotide sequence of said loop comprises:
- a length of between 8 and 24 nucleotides;
- a GC content between 55 and 70%;
- a melting temperature between 60 °C and 70 °C;
and wherein said nucleotide sequence does not:
- comprise more than three consecutive identical nucleotides;
- comprise a deoxycytosine (C) or deoxyguanosine (G) at the 5' end of the loop;
- form a secondary structure;
- show complementarity to the human genome;
- or show complementarity to itself.

In a preferred embodiment, the nucleotide sequence of said loop comprises 18 nucleotides.

In a preferred embodiment, said universal detection probe comprises a molecular beacon probe. Molecular beacons are oligonucleotide probes that contain both fluorophore and quencher moieties and can report the presence of specific nucleic acids in homogeneous solutions.

In an embodiment, the fluorophore of the detection probe is located at the 5 '-end of the universal detection probe, and at least a first quencher moiety is located at the 3 '-end of the universal detection probe.

In some embodiments, the detection probe is at least partially quenched when not hybridized to a complementary sequence in the amplification reaction, and is at least partially unquenched when hybridized to a complementary sequence in the amplification reaction. In some embodiments, the detection probes of the present teachings have a Tm of about 63°C to about 69°C, though it will be appreciated that guided by the present teachings routine experimentation can result in detection probes with other Tms. The probes of the current invention can comprise natural and/or modified nucleotide residues and internucleotide linkages. Alternative nucleic acid backbones can for instance include but are not limited to phosphorothioate, phosphoroselenoate, alkyl phosphotriester, aryl phosphotriester, alkyl phosphonate, aryl phosphonate, phosphoboronate, morpholino nucleic acid (MNA), locked nucleic acids (LNA), peptide nucleic acids (PNA). In an embodiment, the probe comprises a blocking agent at its 5'end in order to block the 5'end. In an embodiment, said blocking agent is a hydrophobic 6-carbon spacer. In some embodiments, probes may further comprise various modifications such as a minor groove binder (see for example U.S. Patent 6,486,308) to further provide desirable thermodynamic characteristics.

In an embodiment, said molecular beacon probes are single-stranded hairpinshaped oligonucleotide probes.

As used herein, the terms "hairpin" and "stem-loop" are interchangeable and are used to indicate the structure of an oligonucleotide in which one or more portions of the oligonucleotide form base pairs with one or more other portions of the oligonucleotide. When the two portions are base paired to form a double-stranded portion of the oligonucleotide, the double-stranded portion may be referred to as a stem.

In the presence of the target sequence, the probe unfolds, binds and emits a signal (e.g., fluoresces).

Such a molecular beacon typically includes at least four components: 1) the "loop", an 8-24 nucleotide region which is complementary to the target sequence; 2) two 5-7 nucleotide "stems" found on either end of the loop and being complementary to one another; 3) at the 5' end, a detectable label (a fluorophore); and 4) at the 3' end, a quencher moiety that prevents the detectable label from emitting a signal when the probe is in the closed loop shape (e.g., not bound to a target nucleic acid). Thus, in the presence of a complementary target, the "stem" portion of the beacon separates out resulting in the probe hybridizing to the target. Other types of molecular beacons are also known and may be suitable for use in the PCR detection systems and methods described herein. In a hairpin configuration, the nucleotides in the stem show a 5'-3' versus 3'-5' hybridization.

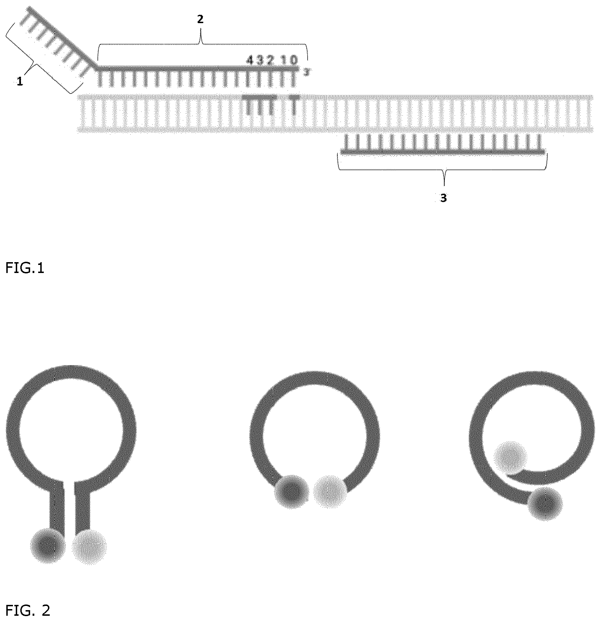

In another embodiment, said molecular beacon probe has a shell form, wherein the nucleotides in the stem show a 5'-3' versus 5'-3' stem hybridization. In yet another embodiment, said molecular beacon probe does not comprise a stem, also referred to as a "horseshoe" probe. Examples of such configurations are depicted in figure 2 (hairpin (left), horseshoe (middle), shell (right)). The probes comprise a fluorophore at one end (for instance, at the 5' end) and at the other end (in this case, the 3' end), a quencher moiety that prevents the fluorophore from emitting a signal when the probe is in the closed loop shape (e.g., not bound to a target nucleic acid).

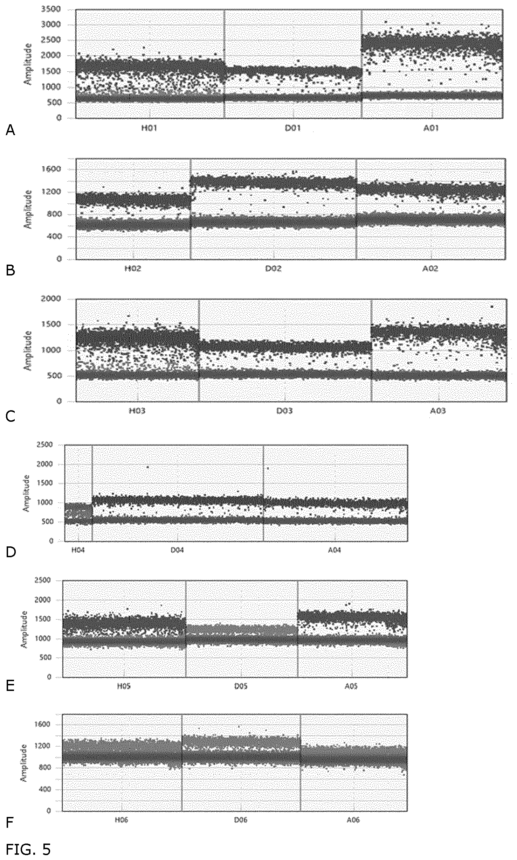

In an embodiment, the stem of the molecular beacon probe consists of 4, 5, 6, 7, 8, 9 or 10 nucleotides. The inventors performed experiments to determine an optimal stem length (see example 1.3 and figure 5). Based on maximal cluster separability, an optimal stem length of 5 nucleotides was selected. In a preferred embodiment, the stem consists of 5 nucleotides.

As such, in an embodiment, said molecular beacon probe comprises a double stranded stem and a single stranded loop, wherein said molecular beacon probe is a hairpin probe, wherein the nucleotides in the stem show a 5'-3' versus 3'-5' hybridization. In another embodiment, said molecular beacon probe comprises a double stranded stem and a single stranded loop, wherein said molecular beacon probe is a shell form probe, wherein the nucleotides in the stem show a 5'-3' versus 5'-3' hybridization. In another embodiment, said molecular beacon probe is stemless.

In a preferred embodiment, said molecular beacon probe comprises a double stranded stem of 5 bp and a single stranded loop of 18 nucleotides.

In a further aspect, the invention relates to use of aforementioned universal detection probe in a PCR detection system or a PCR method according to the current invention, wherein said universal detection probe is used in combination with a universal primer comprising a 5'-universal tail, wherein the nucleotide sequence of said loop allows selective hybridization to the complement of a nucleotide sequence comprised in said 5'-universal tail of said primer.

As described above, the first 5'-universal tail of said first primer comprises a nucleotide sequence, the complement of which allows selective hybridization to a nucleotide sequence comprised in said first universal detection probe.

In an embodiment, the loop (or part of the loop) of the molecular beacon probe comprises the nucleotide sequence which allows selective hybridization to a nucleotide sequence comprised in the 5'-universal tail of the primer.

In an embodiment, the nucleotide sequence of the single stranded loop has at least 90% sequence identity or is identical to a nucleotide sequence comprised in said 5'-universal tail of the primer. In an embodiment, the nucleotide sequence of the single stranded loop has 90%, 91%, 92%, 93%, 94%, 95%, 96%, 97%, 98%, 99%, 100% sequence identity or is identical to a nucleotide sequence comprised in said 5'-universal tail of the primer.

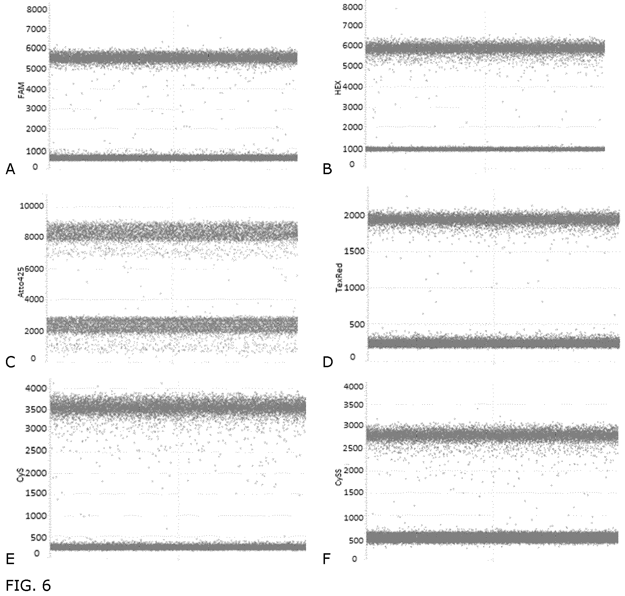

The PCR detection system of the current invention is compatible with any fluorophore (see example 1.4 and figures 6 A-F).

As used herein, a "fluorophore" refers to any fluorescent compound that can reemit light upon light excitation.

In an aspect, a fluorophore is a peptide or protein. In an aspect, a fluorophore is a small organic compound. In an aspect, a fluorophore is a synthetic oligomer or polymer. In an aspect, a fluorophore is a multi-component system. In an aspect, a fluorophore is selected from the group consisting of a peptide or protein, a small organic compound, a synthetic oligomer or polymer, and a multi-component system. In an aspect, a fluorophore is an organic dye.

Non-limiting examples of peptide or protein fluorophores included green fluorescence protein (GFP), yellow fluorescence protein (YFP), and red fluorescence protein (RFP).

Non-limiting examples of small organic compound fluorophores include xanthene derivatives (e.g, fluorescein, rhodamine, Oregon green, eosin, Texas red), cyanine derivatives (e.g. cyanine, indocarbocyanine, merocyanine), squaraine derivatives, squararine rotaxane derivatives, naphthalene derivatives, coumarin derivatives, oxadiazole derivatives, anthracene derivatives, pyrene derivatives (e.g, Cascade blue), oxazine derivatives (e.g, Nile red, Nile blue, cresyl violet), acridine derivatives (e.g, proflavine, acridine orange, acridine yellow), arylmethine derivatives (e.g. auramine, crystal violet, malachite green), tetrapyrrole derivatives, and dipyrromethene derivatives.

In an aspect, a fluorophore is selected from the group consisting of Cyanine 3 (Cy3^{™}), Fluorescein (FAM), 2'-chloro-7'-phenyl-1,4-dichloro-6-carboxyfluorescein (VIC), Hexachlorofluorescein (HEX), Cyanine 5 (Cy5^{™}), Cyanine 5.5 (Cy5.5^{™}), carboxy-X-rhodamine (ROX), ATTO425, Texas Red (TR) and Quasar 705.

In an aspect, a fluorophore is selected from the group consisting of a Freedom^{™} dye, an ATTO^{™} dye, an Alexa Fluor^{®} dye, a LI-COR IRDye^{®}, a Dyomics^{™} dye and a Rhodamine dye.

Quenchers can be used to decrease the fluorescence intensity of a given substance, such as a fluorophore. As used herein, a "quencher" refers to any substance that absorbs the excitation energy from a fluorophore, thereby reducing or eliminating the fluorescence intensity of the fluorophore. In an aspect, a quencher dissipates the light energy from a fluorophore as heat. In an aspect, a quencher is a dye that lacks native fluorescence.

In an aspect, a quencher is selected from the group consisting of MGB, Black Hole Quencher, Iowa Black^{®} RQ, and Iowa Black^{®} FQ.

The inventors performed experiments in order to optimize the concentrations of the unmodified primer, the primer comprising a 5'-universal tail sequence and of the detection probe (see example 3). They concluded that the concentration of the modified primer should be lower than that of the unmodified (regular) primer (asymmetric PCR) to have good separability. Furthermore, probe concentrations are preferably higher than the modified primer concentration. In a preferred embodiment, the concentration of the detection probe is at least somewhat higher than the concentration of the primer comprising the 5'-universal tail sequence. In a preferred embodiment, the concentration of the detection probe is lower than the concentration of the unmodified primer.

In a preferred embodiment, the concentrations of the unmodified primer, the primer comprising a 5'-universal tail sequence and of the detection probe are optimized.

In an embodiment, the PCR reaction comprises 300 nM unmodified primer, 100 nM primer comprising a 5'-universal tail sequence and 125 nM detection probe.

As will be appreciated by those skilled in the art, the oligonucleotides disclosed herein may be used as one or more primers in various extension, synthesis, or amplification reactions.

Primer-dependent amplification reactions comprise repeated thermal cycles of primer annealing, primer extension, and strand denaturation (strand melting). Primer annealing may be performed at a temperature below the primer-extension temperature (for example, three-temperature PCR), or primer annealing and primer extension may be performed at the same temperature (for example, twotemperature PCR). The overall thermal profile of the reaction may include repetitions of a particular cycle, or temperatures/times may be varied during one or more cycles.

The inventors performed experiments to find the optimal thermocycling conditions. The inventors found that longer denaturation and longer elongation results in better separability. In an embodiment, the denaturation step lasts more than 1 second, preferably more than 2 seconds, preferably more than 3 seconds, preferably more than 4 seconds, such as 5 seconds. In an embodiment, the elongation step last more than 1 second, preferably more than 2 seconds, preferably more than 3 seconds, preferably more than 4 seconds, preferably more than 5 seconds, preferably more than 6 seconds, preferably more than 7 seconds, preferably more than 8 seconds, preferably more than 10 seconds, preferably more than 11 seconds, preferably more than 12 seconds, preferably more than 13 seconds, preferably more than 14 seconds, preferably more than 15 seconds, preferably more than 16 seconds, preferably more than 17 seconds, preferably more than 18 seconds, preferably more than 19 seconds, preferably more than 20 seconds, preferably more than 21 seconds, preferably more than 22 seconds, preferably more than 23 seconds, preferably more than 24 seconds, preferably more than 25 seconds, preferably more than 26 seconds, preferably more than 27 seconds, preferably more than 28 seconds, preferably more than 29 seconds, such as 30 seconds.

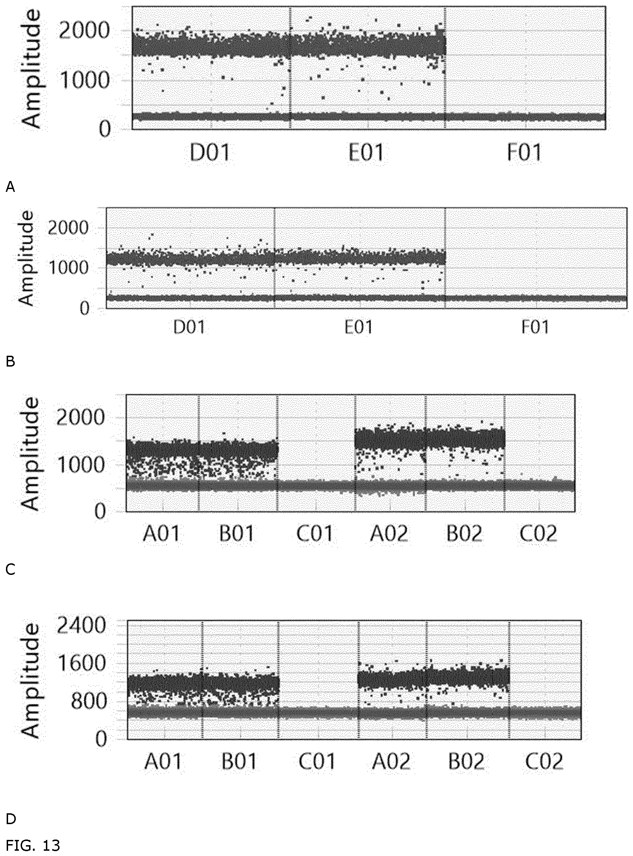

The inventors found that slow cooling after final denaturation at the end of the PCR increases the separability of a digital PCR assay (see Example 4 and figure 13). In an embodiment, the PCR dependent amplification reaction may include a slow cooling at the end until the temperature for imaging is reached (for instance 40°C or room temperature (25°C)). Slow cooling refers to a decrease in temperature after final denaturation at the end of the PCR of less than 4 °C/s, preferably less than 3 °C/s, preferably less than 2 °C/s, preferably less than 1 °C/s, such as 1 °C/s.

Touchdown (TD) PCR is known from the art and offers a simple and rapid means to optimize PCRs, increasing specificity, sensitivity and yield. TD-PCR employs an initial annealing temperature (Ta) above the projected melting temperature (Tm) of the primers being used, then progressively transitions to a lower, more permissive annealing temperature over the course of successive cycles. Any difference in Tm between correct and incorrect annealing will produce an exponential advantage of twofold per cycle. TD-PCR has found wide applicability in standard PCR protocols, including reverse transcriptase-dependent PCR, as well as in the generation of cDNA libraries and single nucleotide polymorphism screening. TD-PCR is particularly useful for templates that are difficult to amplify but can also be standardly used to enhance specificity and product formation. The procedure takes between 90 and 120 min, depending on the template length.

The inventors surprisingly found that when such a 'touchdown' protocol is followed by a 'touch-up' (TU) step the selectivity of the PCR reaction is even more increased. In an embodiment, a touchdown protocol comprises decreasing the annealing temperature (Ta) 1 °C/cycle when starting at a conventional Ta + n °C (for n cycles) until Ta is reached and the 'touch-up' protocol comprises increasing the annealing temperature 1 °C/cycle for n/2 cycles until Ta + n/2 °C is reached, followed by remaining cycles at Ta + n/2 °C. For instance, in an embodiment, the temperature is decreased over 10 cycles from 70 °C to 60 °C (TD), increased again to 65 °C over 5 cycles and kept at 65 °C for the remainder of the PCR program (TU).

Nucleic acid analysis techniques that identify alterations or polymorphisms within known sequences are useful in many aspects of scientific, medical and forensic fields. For example, these techniques can be used in the genotyping of individuals in order to diagnose hereditary diseases or provide prognosis based on known genetic lesions. Genotypes of interest include, for example, point mutations, deletions, insertions and inversions as well as polymorphisms within nucleic acid sequences of interest. These techniques can also be used for clinical purposes such as tissue typing for histocompatability or for forensic purposes such as identity or paternity determination.

Furthermore, nucleic acid analysis techniques can be used for the identification of organisms or to distinguish or identify pathogenic organisms of infectious agents. In addition, these techniques are useful in the identification and monitoring of genetically modified agricultural organisms such as crops and livestock.

A single nucleotide variant (SNV) is a variation of a single nucleotide in a population's genome. Like SNVs, a single nucleotide polymorphism (SNP) is also a single base substitution, but it is limited to germline DNA and must be present in at least 1% of the population.

SNPs are an abundant form of genetic variation. These single nucleotide changes are found approximately every 500 bp in the human genome. Almost all SNPs are bi-allelic, that is, only two different alleles exist. Typically, one allele is present in the majority of the chromosomes of a population, and the alternative variant, that is, the minor allele, is present with less frequency. Only alleles that are present at a frequency greater than 1% are considered polymorphisms.

As used herein, the term "single nucleotide polymorphism" (SNP) refers to a DNA sequence variation occurring when a single nucleotide, e.g., A, T, C, or G, in the genome (or other shared sequence) differs between members of a species (or between paired chromosomes in an individual). For example, two sequenced DNA fragments from different individuals, AAGCCTA to AAGCTTA contain a difference in a single nucleotide. In this case, it is to be understood that there are two alleles: C and T. Almost all common SNPs have only two alleles.

SNPs are promising tools for mapping susceptibility mutations that contribute to complex diseases. Although most SNPs are neutral and do not affect phenotype, they can be used as surrogate markers for positional cloning of genetic loci, because of the allele association, known as linkage disequilibrium, that can be shared by groups of adjacent SNPs. A need exists for alternative methods that give high performance and sequence specificity necessary to correctly identify a unique organism, disease state or clinical condition of interest that also have a simplified workflow, lower cost, and/or faster turn-around time.

When a sequence variant (e.g. SNP, SNV, or any somatic mutation) must be quantified, an allele-specific primer is needed.

As such, according to certain embodiments, the amplification of nucleic acid sequences may involve the use of locus-specific and/or allele-specific primers.

Locus-specific primers (LSPs) may be used to amplify all alleles encoded at a given locus, but not all alleles encoded by other loci.

Allele-specific primers (ASPs) are LSPs that amplify families of alleles that share a common polymorphism. Allele-specific primers may be used to amplify a single allele and may be capable of differentiating between two sequences that differ by only a single base difference of change.

As such, in an embodiment said first primer (being modified and having a first 5' universal tail) is a first allele-specific primer, which is specific for a first allele of the target nucleic acid.

As used herein, the term "allele-specific primer" or "ASP" refers to a primer that binds to a specific sequence on a region of a nucleic acid to be amplified. These types of primers may be used to amplify and discriminate between two or more alleles of a gene, for example, simultaneously. The difference between the two alleles may be a SNP, a SNV, an insertion, a deletion, or a sequence difference generated by bisulfite conversion of unmethylated DNA.

In a preferred embodiment, the allele-specific primer ends at its 3' ultimate position at the variant nucleotide position.

Such allele-specific primers of the PCR detection system of the current invention include an allele-specific portion and a 5'-universal tail; wherein the 5'- universal tail is not complementary to the target nucleic acid sequence. The 5'-universal tail includes a nucleotide sequence the complement of which allows hybridization to a sequence comprised in said first universal probe sequence. Figure 1 shows such a allele-specific primer having a 5'-universal tail (depicted as number 1 in the figure) and a region configured to selectively hybridize to said target nucleic acid under amplification conditions (depicted as number 2 in the figure). Number 3 depicts the unmodified reverse primer.

In certain embodiments, the allele-specific primer (including both the 5'-universal tail and a region configured to selectively hybridize to said target nucleic acid) has a length of about 26 nucleotides to about 55 nucleotides. Preferably, the allele-specific primer has a length of about 35 nucleotides to about 50 nucleotides. Most preferably, the allele-specific primer has a length of about 36-43 nucleotides. In certain embodiments, the allele-specific portion has a Tm of about 53 °C to about 65 °C, preferably about 58 °C to about 62 °C, most preferably about 59 °C.

As described above, said 5'-universal tail of said first allele-specific primer can comprises a nucleotide sequence having a sequence identity of at least 90%, at least 91%, at least 92%, at least 93%, at least 94%, at least 95%, at least 96%, at least 97%, at least 98% or at least 99% to a nucleotide sequence comprised in said first universal detection probe, wherein said nucleotide sequence showing sequence identity comprises at least 8 nucleotides. As described above, said nucleotide sequence showing sequence identity can consists of 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides or 30 nucleotides. In a preferred embodiment, said nucleotide sequence showing sequence identity consists of between 15 and 20 nucleotides, such as 18 nucleotides.

In a further preferred embodiment, said 5'-universal tail of said first allele-specific primer comprises a nucleotide sequence which is a 100% identical to a sequence comprised in said first universal detection probe, wherein said identical nucleotide sequence comprises at least 8 nucleotides. Preferably said identical nucleotide sequence consist of 18 nucleotides.

In an embodiment, the PCR detection system further comprises:
a. a second primer, wherein said second primer is a second allele-specific primer, which is specific for a second allele of the target nucleic acid and wherein said second primer comprises a region configured to hybridize to said target nucleic acid under amplification conditions and a second 5'-universal tail which is not complementary to the target nucleic acid sequence;
b. a second universal detection probe, wherein said second universal detection probe comprises a second fluorophore, wherein said second 5'-universal tail of said second primer comprises a nucleotide sequence, the complement of which allows hybridization to a nucleotide sequence comprised in said second universal detection probe and wherein the first and second fluorophore are different.

As such, in a further aspect, the invention also relates to a PCR method for determining the genotype of a target nucleic acid molecule by means of aforementioned PCR detection system, the method comprising: providing a reaction mixture comprising one or more target nucleic acid molecules and a first allele-specific primer which is specific for a first allele, said first allele-specific primer comprising a first 5'-universal tail which comprises a nucleotide sequence, the complement of which allows hybridization to a nucleotide sequence comprised in a first universal detection probe, wherein the first universal detection probe comprises a first fluorophore; and a second allele-specific primer which is specific for a second allele, said second allele-specific primer comprising a second 5'-universal tail which comprises a nucleotide sequence the complement of which allows hybridization to a nucleotide sequence comprised in a second universal detection probe, wherein the second universal probe comprises a second fluorophore; wherein the first and second fluorophore are different; amplifying the target nucleic acids; measuring the intensities of the first and the second fluorophore; and analyzing nucleic acid genotypes based on the intensities of the first and second fluorophores.

By providing a second primer which is specific for a second allele and which has a second 5'-universal tail, a second universal detection probe having a second fluorophore can bind to the complement sequence of the second 5'-universal tail. As such, the second allele of the target nucleic acid can be analyzed, quantitated or detected in the same PCR reaction. For instance, by providing a specific primer for the wild-type and for the mutant allele, both alleles can analyzed, quantitated or detected in the same PCR reaction

Likewise to the 5'-universal tail of said first primer described above, said 5'-universal tail of said second primer can comprises a nucleotide sequence having a sequence identity of at least 90%, at least 91%, at least 92%, at least 93%, at least 94%, at least 95%, at least 96%, at least 97%, at least 98% or at least 99% to a nucleotide sequence comprised in said second universal detection probe, wherein said nucleotide sequence showing sequence identity comprises at least 8 nucleotides. Likewise to the 5'-universal tail of said first primer described above, said nucleotide sequence showing sequence identity can for instance consists of 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides or 30 nucleotides. In a preferred embodiment, said nucleotide sequence showing sequence identity consists of between 15 and 20 nucleotides, such as 18 nucleotides.

In a further preferred embodiment, said 5'-universal tail of said second primer comprises a nucleotide sequence which is a 100% identical to a sequence comprised in said second universal detection probe, wherein said identical nucleotide sequence comprises at least 8 nucleotides. Preferably said identical nucleotide sequence consist of 18 nucleotides.

As described above, in a preferred embodiment, the allele-specific primer ends at its 3' ultimate position at the variant nucleotide position, having a 3'-terminal interrogating nucleotide, also known as an ARMS (Amplification Refractory Mutation System) primer. An ARMS primer is a primer that is allele-discriminating, because its 3'-terminal nucleotide is an interrogating nucleotide.

However, the selectivity of such a primer is not always good enough.

As such, in an embodiment, a destabilized primer can be used. One way of destabilizing a primer (and making it more selective) is by interrupting the primer by a non-complementary bridge (or loop). Another way is by introducing an artificial mismatch somewhere in the 3' end of the primer. For instance, an ARMS primer may include a deliberately introduced nucleotide near its 3' end that is mismatched both to the intended target sequence and to the unintended target sequence to destabilize the primer and increase its allele discrimination. In a preferred embodiment, the mismatched nucleotide can occur at positions 1, 2, 3 or 4, with the interrogating nucleotide at position 0 (see Figure 1).

As used herein, a "mismatch" refers to an alignment of two sequences that pairs two uncomplimentary nucleotides. Non-limiting examples of mismatches include G-A, G- T, G-U, G-G, C-A, C-T, C-U, C-C, A-A, T-T, and T-U. Conversely, "matched" alignments of nucleotides refer to complimentary pairs such as G-C, A-T, and A-U.

As such, in an embodiment, said first allele-specific primer and/or said second allele-specific primer may further include a deliberately introduced nucleotide near its 3' end in the region configured to hybridize to said target nucleic acid that is mismatched to the target nucleic acid. In an embodiment, the deliberately introduced nucleotide is at position 1, 2, 3 or 4 as depicted in Figure 1 (having the interrogating nucleotide at position 0). By introducing such a mismatch, the primer is destabilized and its allele discrimination is increased.

A specific preferred embodiment of the current invention relates to a PCR detection system, wherein said first allele-specific primer and said second allele-specific primer each include a deliberately introduced nucleotide near its 3' end in the respective regions that is mismatched to the target nucleic acid and wherein said mismatched nucleotide is different in said first allele-specific primer and said second allele-specific primer. Said mismatched nucleotides might differ with their mismatch position or sequence.

As such, when the natural template is amplified, the allele-matched primer has 1 mismatch relative to its target (i.e. the artificially induced mismatch) and the allele-non-matched primer has 2 mismatches relative to its off-target (i.e. the artificially induced mismatch and the 3' end ultimate position mismatch). As a result, on the generated amplicons, the allele-specific primers have 0 mismatches to their respective targets, and 3 mismatches to the off-target amplicons (making them effectively unamplifiable).

The unique benefit is that generated amplicons for one allele cannot be used as template for the off-target primer (because there are 3 mismatches, see table 1 below). "WT" refers to wild type, "MUT" refers to mutant, "MM" refers to mismatch.

**Table 1**

| | | MM between primer and template with MM at same position | | MM between primer and template with MM at different position | |
|---|---|---|---|---|---|
| primer | template | cycle 1 | cycle 2-50 | cycle 1 | cycle 2-50 |
| WT | WT | 1 | 0 | 1 | 0 |
| | MUT | 2 | 1 | 2 | 3 |
| MUT | WT | 2 | 1 | 2 | 3 |
| | MUT | 1 | 0 | 1 | 0 |

By introducing a mismatch in both the first allele-specific primer and the second allele-specific primer, wherein the mismatched nucleotide is different in said first allele-specific primer and said second allele-specific primer, one can increasing the genotype discriminatory power of the PCR method.

As such, the invention further relates to a specific use of the PCR detection system according to the current invention for increasing the genotype discriminatory power of the PCR method.

Selectivity can be further enhanced by optimizing the PCR conditions using known strategies (annealing temperature, cycle duration, Tm modifying reagents (e.g. DMSO, TMAC), Tm modifying nucleotides in the primer (e.g. LNA), special thermocycling conditions.

In order to increase the selectivity further, the reaction mixture may further includes a selectivity-enhancing reagent, such as a Hofmeister salt. In an embodiment, the Hofmeister salt comprises tetramethylammonium chloride (TMAC).

In an embodiment, selectivity is enhanced by providing allele-specific primers for both the forward and the reverse primer, wherein both the forward and the reverse primer end with the 3' ultimate nucleotide at the nucleotide variant position.

Such a PCR detection system having allele-specific primers for both the forward and the reverse primer is compatible with the current invention as the universal detection probes of the current invention, don't require any target sequence. Furthermore, it prevents polymerase induced errors at the nucleotide site of interrogation. Polymerase errors during PCR are considered one of the major sources of false-positive results.

As described above, oligonucleotides are provided that are specific for a particular locus of the target nucleic acid. Such oligonucleotides are referred to herein as locus-specific primers or LSPs. In certain embodiments, the LSP may also be specific to a particular allele and is known as an allele-specific primer.

In certain embodiments, the unmodified primer is a locus-specific primer and may serve as a reverse primer for both alleles in the amplification and genotyping methods disclosed herein. In certain embodiments, the unmodified primer is a locus-specific primer and may serve as a forward primer for both alleles in the amplification and genotyping methods disclosed herein.

In an embodiment, wherein said first and/or second allele-specific primers having a 5'-universal tail are forward primers, the reaction mixture further comprises a locus specific unmodified reverse primer. In an alternative embodiment, wherein said first and/or second allele-specific primers having a 5'-universal tail are reverse primers, the reaction mixture further comprises a locus specific unmodified forward primer.

In an embodiment, the primer pair (forward and reverse primer) exist of allele-specific primers, of which one is modified at its 5' end. When both the forward and reverse primer is allele-specific, the selectivity of the reaction is enhanced.

In an embodiment, wherein said first and/or second allele-specific primers having a 5'-universal tail are forward primers, the reaction mixture may further comprise a first and second allele-specific reverse primer.

As such, in an embodiment, wherein said first and/or second allele-specific primers having a 5'-universal tail are reverse primers, the reaction mixture may further comprise a first and second allele-specific forward primer.

In certain embodiments, an amplification reaction comprises multiplex amplification, in which a multiplicity of different target nucleic acids and/or a multiplicity of different amplification product species are simultaneously amplified using a multiplicity of different primer sets. Multiple different spectrally distinct fluorophores can be used on different probes to allows multiplexed detection and quantitation of multiple distinct DNA target species in a single reaction.

As such, in an embodiment, the method of the current invention further comprises simultaneously quantifying one or more further target nucleic acid molecules in a multiplex reaction, thereby providing one or more further primers which are specific for the one or more further target nucleic acid molecules, said one or more further primers comprising one or more further 5'-universal tails which comprise a nucleotide sequence the complement of which allows hybridization to a nucleotide sequence comprised in one or more further universal detection probes, wherein each of said one or more further universal detection probes comprises a further fluorophore; wherein the further fluorophores are different; amplifying the target nucleic acids; measuring the intensities of the fluorophores; and quantifying the target nucleic acid molecules based on the intensities of the fluorophores.

When allele-specific primers are used, the method of the current invention further allows to determine the genotype of one or more further target nucleic acid molecules in a multiplex reaction.

As such in a further embodiment, the method of the current invention further comprises simultaneously determining the genotype of one or more further target nucleic acid molecules in a multiplex reaction, thereby providing one or more further allele-specific primers which are specific for the one or more further alleles of the one or more further target nucleic acid molecules, said one or more further allele-specific primers comprising one or more further 5'-universal tails which comprise a nucleotide sequence the complement of which allows hybridization to a nucleotide sequence comprised in the one or more further universal probes, wherein each of said one or more further universal probes comprises a further fluorophore; wherein the further fluorophore is different from the first and second fluorophore; amplifying the target nucleic acids; measuring the intensities of the first, the second fluorophore and the one or more further fluorophores; and analyzing nucleic acid genotypes based on the intensities of the first, second and one or more further fluorophores.

In a further aspect, the invention relates to use of a PCR detection system as described above for analyzing, quantitating or detecting one or more alleles or polymorphisms in a target nucleic acid.

In a preferred embodiment, the PCR reaction is a digital PCR assay, including assays carried out in many different reaction wells in a thermal cycler, and droplet digital PCR (ddPCR) assays carried out in many different droplets in a thermal cycler; and in both cases detection of the resulting amplicons is often carried out in a separate detection instrument, for example the Bio-Rad QX200^{™} Droplet Digital PCR System, the Qiagen QIAcuity digital PCR or the Stilla Technologies Naica^{™} System.

Such methods utilize for each of the numerous target sequences a universal primer that has a 5'-tag (or 5'-tail) sequence that is different from all other 5'-tag sequences in the reaction. For each of the different target sequences there is a probe that targets the complement of its 5'-tag sequence, allowing to detect the different target sequences in the same PCR reaction (known as multiplexing). Because digital PCR includes subdividing a reaction mixture into so many wells or droplets that each well or droplet is very likely to contain only one target molecule or no target molecule at all, such methods are quantitative.

The basic principal underlying digital PCR assays (illustrated here by a description of ddPCR) is that a sample can be diluted to such an extent that only one target DNA molecule is present in a droplet (or no target molecule is present in a droplet), and there are a large number of droplets. Then, simultaneous PCR amplifications are carried out in each droplet, and fluorescently labeled probes that are present in each droplet bind to the amplicons generated in that droplet (if it contained a target molecule), and become brightly fluorescent in a particular color code, indicating both that the droplet contained a target molecule and identifying which target sequence that was. The number of droplets that light up in the same color code provides an accurate measure of the number of the corresponding target molecules in the original sample; and this approach is so sensitive that even a single target molecule in a sample can be detected. Classical droplet digital PCR has been used to detect and quantitate rare somatic mutations relevant to cancer diagnosis, prognosis, and therapy. In such digital PCR assays, detection may occur in a thermal cycler, in a flow cytometer, or with a microscope.

Another objective of this invention is to provide an amplitude based multiplexing assay method using the PCR detection system according to the current invention.

Amplitude based multiplexing allows to detect the same fluorophore having different amplitudes in the same channel, thereby increasing the number of targets that can be detected in the same fluorescent detection channel.

In order to achieve this the reaction mixture may further comprise one or more further primers which comprise a region configured to selectively hybridize to said target nucleic acid under amplification conditions, but wherein said one or more further primers do not include a 5'-universal tail.

The reaction mixture may also comprises one or more further primers which comprise a region configured to hybridize to said target nucleic acid under amplification conditions, wherein said one or more further primers comprise a further 5'-universal tail, but wherein the reaction mixture does not comprise a further universal detection probe comprising a nucleotide sequence allowing selective hybridization of the further universal detection probe to the complement of the further 5'-universal tail. This concept is known as the inclusion of a dark primer.

This is another approach to enable amplitude multiplexing or dampen the fluorescent signal. This dark primer is modified with a tail that is not compatible with any of the probes used. By adding a dark primer as a fraction of the total target specific primer concentration (e.g., 1:4, 2:4, or 3:4), the dark primer will compete with the probe-compatible modified primer resulting in a lower endpoint fluorescence of the particular target. The higher the fraction of the dark primer, the higher the competition and the lower the fluorescence.

By including further primers that do not comprise a 5'-universal tail or by including primers that do include a 5'-universal tail, but wherein the reaction mixture does not comprise a further universal detection probe, the amplitude of the fluorescent signal emitted by a fluorophore of a molecular beacon probe directed to the same target nucleic acid can be modified.

In an embodiment, in order to modulate the amplitude of the fluorescent signal (for instance in a multiplex reaction), one can modulate the concentration of the primer having the 5' universal tail. In an embodiment, one can for instance provide double or half of the optimal primer concentration (for instance double or half of 100 nM).

In an embodiment, in order to modulate the amplitude of the fluorescent signal (for instance in a multiplex reaction), one can modulate the concentration of the universal detection probe. In an embodiment, one can for instance provide double or half of the optimal detection probe concentration (for instance double or half of 125 nM).

In an embodiment, in order to modulate the amplitude of the fluorescent signal (for instance in a multiplex reaction), one can modulate the concentration of the primer having the 5' universal tail, the unmodified primer and/or the universal detection probe.

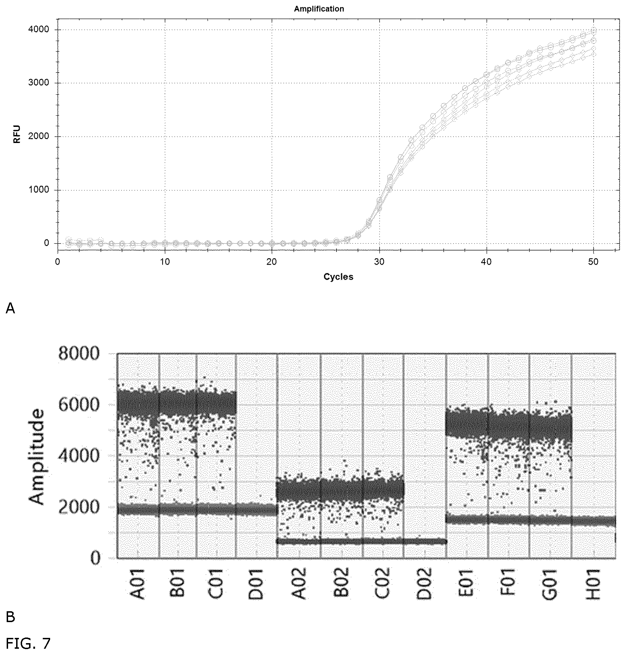

Furthermore, combined use of hairpin and non-hairpin probes (e.g. shell) may facilitate amplitude-based multiplexing (see for instance figure 7B). In this example, the shell probes have a much higher endpoint fluorescence, such that a threshold can be set below the blue positive clusters of shell probes, but higher than the blue positive cluster of the hairpin probe, enabling the discrimination of both clusters in the same channel.

In an embodiment, the invention relates to a specific use of the PCR detection system according to the current invention for performing an amplitude based multiplexing reaction.

Another objective of this invention is to provide a multiplex assay method capable of detecting more target sequences (for example, 12, 20 or 35) than the number of colors the detection instrument can distinguish (the number of detection channels, n).

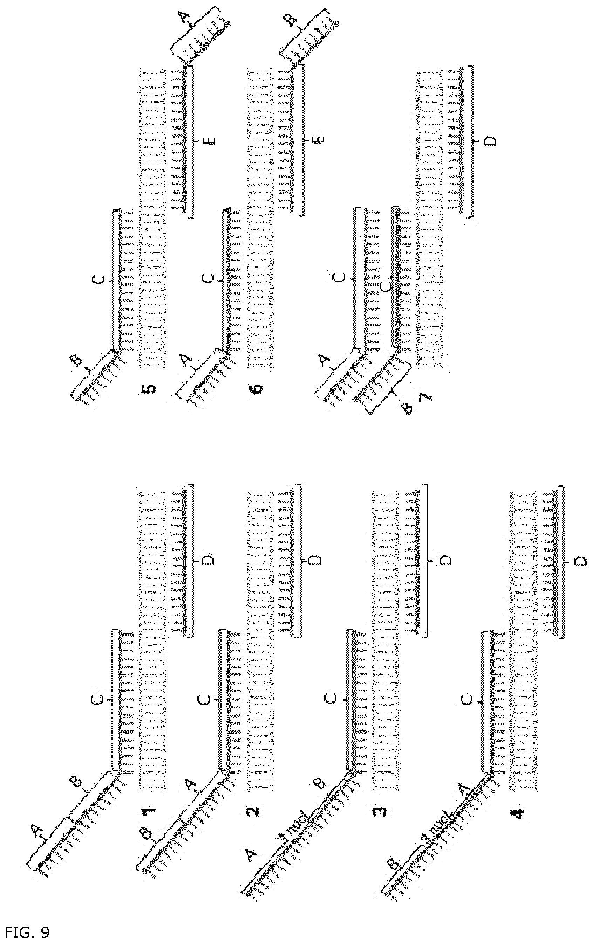

Such higher-order multiplexing can be achieved by using dual-color combinations. With dual-color combinations, n x (n-1)/2 targets can be quantified. By using dual-color combinations, one can detect a single target with two different probes in two different detection channels. The inventors found that good separability can be achieved by modifying the same primer twice with two different probe modifications, resulting in an assay with three primers (2 modified primers, one unmodified (regular) primer) (see example 2 and figures 9-11).

The invention is further described by the following non-limiting examples which further illustrate the invention, and are not intended to, nor should they be interpreted to, limit the scope of the invention.

The present invention will be now described in more details, referring to examples that are not limitative.

### EXAMPLES AND/OR DESCRIPTION OF FIGURES

### Example 1: design of universal detection probes

### 1.1 Loop sequence

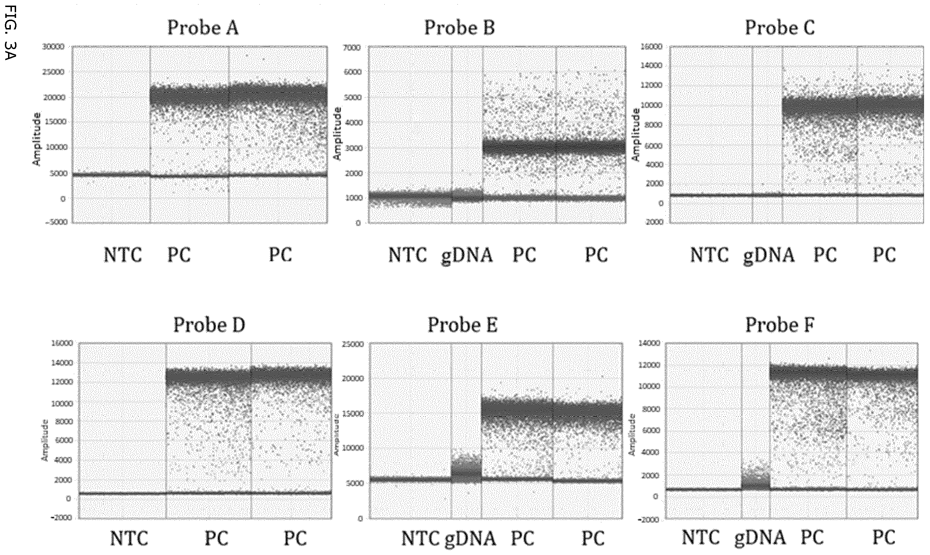

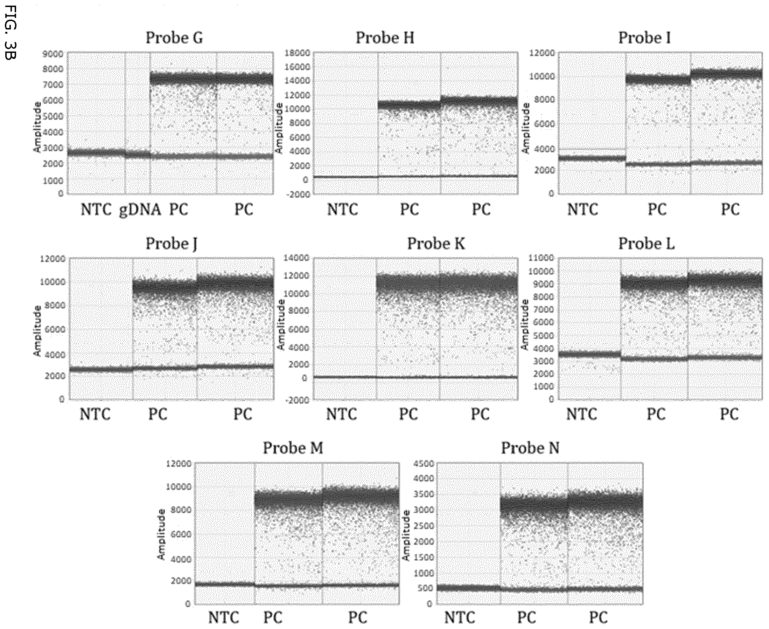

Initially, hairpin-style probes were tested, consisting of a 13-mer loop sequence and a 5-mer stem sequence. The loop sequence is reverse complement sequence of a 5' tail modified primer so amplicons are detected. Fourteen 13-mer loop sequences (A-N) were designed and empirically evaluated. Based on best performance (negative NTC (no-template-control), no signal on human genomic DNA (gDNA), little or no rain (intermediate fluorescent partitions) and a single positive population at the expected concentration), seven 13-mer sequences were selected (A, F, G, H, I, M, N, see Figure 3 A-B), and based on results from point 1.2 (see below, loop length modification), extended and optionally modified into 18-mer probes (capital nucleotides are different from original 13-mer). By increasing the loop length, unwanted signal of the 13-mer occasionally seen on gDNA was absent in the 18-mer version, making peak resolution the primary selection criterium (calculated as described in: Measuring Digital PCR Quality: Performance Parameters and Their Optimization, Lievens A, Jacchia S, Kagkli D, Savini C, Querci M (2016), PLOS ONE 11(5): e0153317. https://doi.org/10.1371/journal.pone.0153317). gDNA samples for some probes failed (partition formation issue) and were repeated on a separate run (data not shown).

### 1.2 Loop length

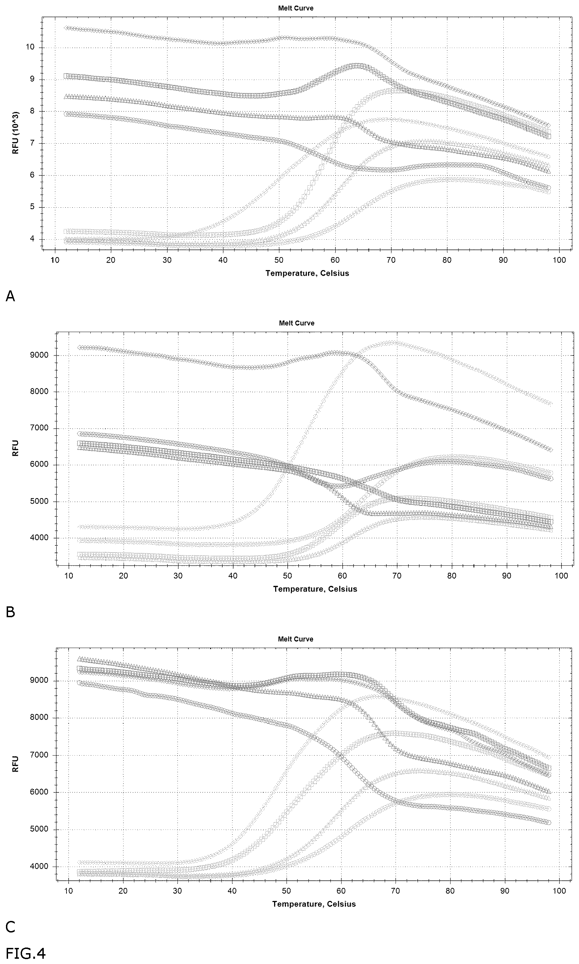

From the same core 13-mer loop sequence, longer versions are generated by extending the loop sequence on the 5' and/or 3' end. Sequences were further optimized to normalize GC-content, melting temperature, and avoid secondary structure formation. Melting curves of PCR amplicons are shown in Figures 4 A-C with the different probes (1, 2 and 3, respectively Figure 4A, 4B and 4C) and different loop lengths 13 (circle), 18 (triangle), 21 (square) and 24 (diamond) for a MYCN target gene assay using DNA as input (highest 4 lines) and NTC (lowest 4 lines).

Data for another assay (BIRC5) is included in table 2 below.

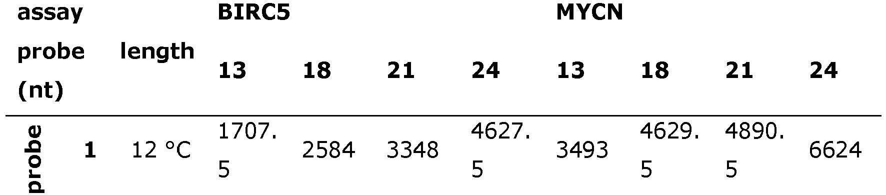

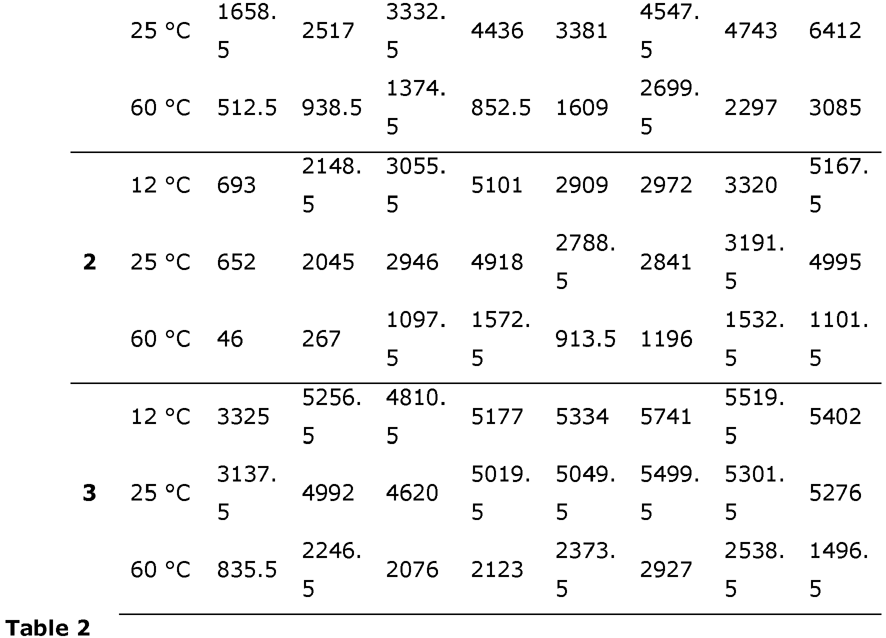

Table 2 lists the difference in RFU values (from a melting curve analysis) between reaction product using either gDNA or NTC measured at different temperatures for 2 assays (BIRC5 and MYCN) for 3 different probes (1, 2 and 3) for 4 different lengths (13, 18, 21 and 24 nt).

Regardless of the loop length, the NTC (negative control) can be differentiated from the gDNA (positive control). The longer the loop, the greater the difference resulting in a better signal-to-noise. A loop length of 18 nt was selected as optimal as longer would also increase the length of the modified primer (shorter primers are preferred to minimize complexity and cross-reactivity).

### 1.3 Stem length and stem sequence

Results for three different stem lengths (5, 6, and 7 nts) and sequences using loop A are shown for a MYCN (**Figure 5** **A, C, E** respectively) and BIRC5 assay (**Figure 5** **B, D, F** respectively). Annealing temperature was tested at 50, 55 or 60 °C (left, middle and right panel). Based on maximal cluster separability, an optimal stem length of 5 nt was selected.

### 1.4 Probe compatibility with any fluorophore

For this experiment, optimal probes were used with a loop of 18 nucleotides and a stem of 5 nucleotides. The same primer (for the wild-type BRAF gene) was modified at its 5' end to be detected by 6 different probes (different loop sequence and different fluorophore) in separate digital PCR reactions using human gDNA as input. See Figures 6A-F. Fluorophores were respectively Fluorescein (FAM), Hexachlorofluorescein (HEX), ATTO425, Texas Red (TR), Cyanine 5 (Cy5), Cyanine 5.5 (Cy5.5). This shows that a given PCR primer sequence can be easily modified, without optimization, for detection using the universal probes.

### 1.5 Non-hairpin probe types (horseshoe and shell)

Other types of probes, different from the stem-containing hairpin probes, are possible. We coin these shell and horseshoe probes. Shell probes have a reverse complementary stem and horseshoe probes have a non-complementary stem (or no stem).

**Figure 2** shows a schematic representation of three possible configurations (hairpin (left), horseshoe (middle), shell (right)) of molecular beacon probes having a fluorophore and a quencher according to an embodiment of the present invention. Hairpin probe (left) with 5'-3//3'-5' complementary 5 bp stem (classic hybridization rules) vs. 'shell' probe (right) whereby there is 5'-3//5'-3' complementary. For example the hairpin probe can have the following configuration: 5'-fluorophore-CCACG-loop-CGTGG-quencher-3'; whereas the shell probe can have the following configuration: 5'-fluorophore-CCACG-loop-GGTGC-quencher-3'.

**Figure 7A** shows the results of two shell probes (Shell_B (diamond) and shell Y (circles)) to detect human genomic DNA on a CFX384 qPCR system. Shell probes work better in qPCR than stem-loop hairpin probes (higher RFU).

**Figure 7B** shows results for Shell_B (A01 to C01), hairpin_B (A02 to C02) and shell_Y (E01 to G01) on human gDNA with their repective no-template-controls (D01, D02 and H01). Shell probes have a much higher endpoint fluorescence and only slighter higher background fluorescence, which results in a better peak resolution or separability (from 3.5 to 4.5). Better separability allows more robust and more accurate thresholding and discriminating negative from positive partitions in digital PCR. Combined use of hairpin and non-hairpin probes (e.g. shell) may facilitate amplitude-based multiplexing. In this example, a threshold can be set below the blue positive clusters of shell probes, but higher than the blue positive cluster of the hairpin probe, enabling the discrimination of both clusters in the same channel.

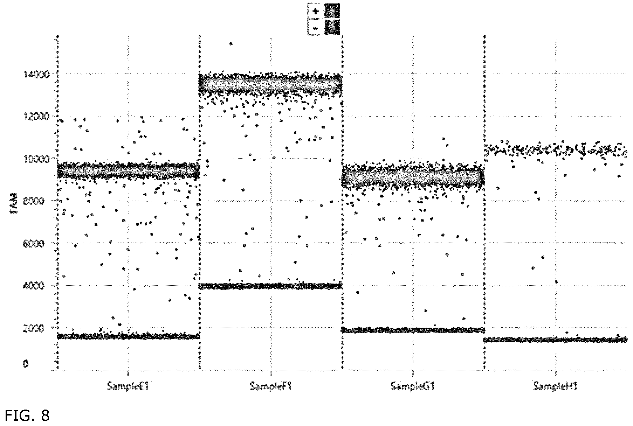

**Figure 8** shows results for 4 different probes tested (see Table 3 below).

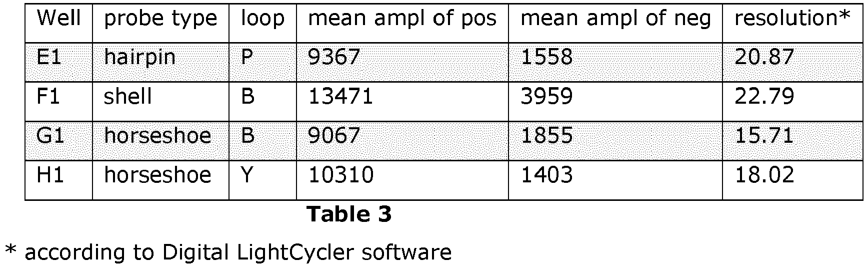

### Example 2: Dual-color combination for higher order multiplexing

We devised seven strategies (see **Figure 9**) to detect a single target with two different probes in two different detection channels:
1. Use a longer primer tail (nucleotide sequence A + nucleotide sequence B) to be compatible with two different probes.
2. Similar to option 1 but switch the order of the modifications (nucleotide sequence B + nucleotide sequence A).
3. Similar to option 2 but add a stuffer of 3 random nucleotides to decrease the possibility of steric hindrance (nucleotide sequence A + stuffer+ nucleotide sequence B).
4. Similar to option 3 but switch the order of the modifications (nucleotide sequence B +stuffer+ nucleotide sequence A).
5. Modify both the forward and the reverse primer as primers.
6. Similar to option 5 but switch the order of the modifications.
7. Modify the same primer twice with two different probe modifications, resulting in an assay with three primers (2 modified primers, one unmodified (common) primer).

Nucleotide sequence C = region of the modified forward primer that is configured to selectively hybridize to the target nucleic acid under amplification conditions. Nucleotide sequence D = unmodified reverse primer. Nucleotide sequence E = region of the modified reverse primer that is configured to selectively hybridize to the target nucleic acid under amplification conditions.

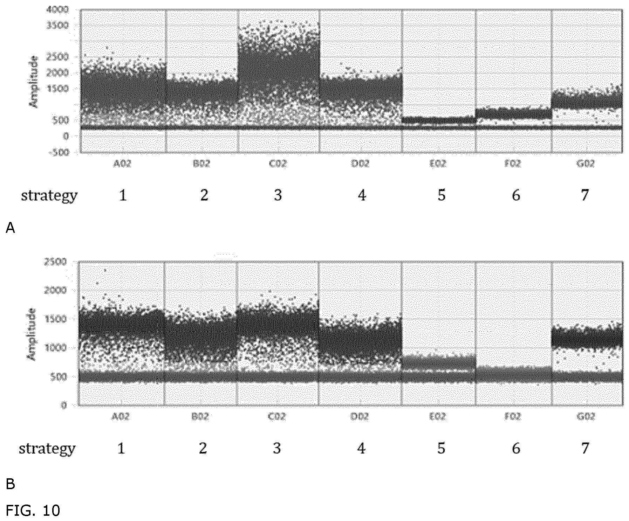

Figure 10A-B show 1D plots for all 7 strategies with probes PXL-RBO-001-P-FAM (A) and PXL-RBO-001-B-HEX (B) for target BRAFV600E_WT. Only strategy 7 results in good separability.

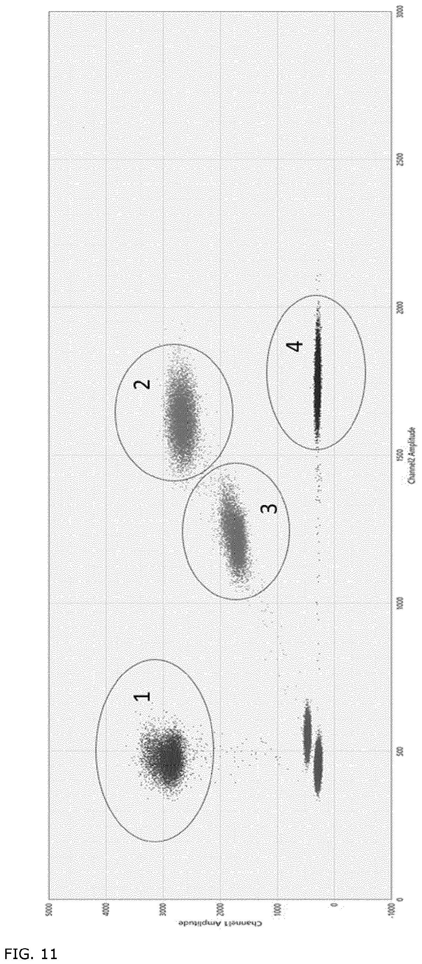

**Figure 11** shows a 2D scatterplot of strategy 7 with PXL-RBO-001-P-FAM (y-axis) and PXL-RBO-001-B-HEX (x-axis), with 1x probe concentration (125 nM, cloud 3) and 2x probe concentration (250 nM, cloud 2). Doubling the probe concentration greatly increases the separability for dual color combination detection. For visualization purposes, the same target was also detected in separate reactions using single probe (PXL-RBO-001-P-FAM (cloud 1) and PXL-RBO-001-B-HEX (cloud 4). Doubling the probe concentration in a dual-color combination assay results in double-positive partitions that have almost the same intensity as the single-color single-positive targets.

### Example 3: optimization of primer and probe concentrations

In this example, primer and probe concentrations of a PCR detection system according to an embodiment of the current invention are optimized. The primer pair (forward plus reverse primer) consists of one primer having a 5'-universal tail ("MOD", "modified primer") and one "unmodified primer" or "regular primer" ("REG") not having such a 5'-universal tail. Table 4 shows an overview of modified primer (MOD), regular primer (REG) and probe (probe) concentrations with their resulting total number of partitions, number of positive partitions and peak resolution. Peak resolution is calculated as described in Lievens *et al.* (2016). The "modified primer" according to an embodiment of the invention comprises a region configured to selectively hybridize to a target nucleic acid under amplification conditions and a 5'-universal tail which is not complementary to the target nucleic acid sequence but comprises a nucleotide sequence, the complement of which allows selective hybridization to a nucleotide sequence comprised in the probe.

**Table 4**

| | | | | total # of partitio ns | # of positive partitio ns | peak resolutio n |
|---|---|---|---|---|---|---|
| ratio (MOD/REG) | MOD (nM) | REG (nM) | probe (nM) | | | |
| 1 | 250 | 250 | 250 | 15757 | 8117 | 1.48 |
| 1/2 | 250 | 500 | 250 | 16944 | 8651 | 5.70 |
| 1/10 | 50 | 500 | 250 | 16292 | 8566 | 4.50 |
| 1/50 | 10 | 500 | 250 | 16032 | 7270 | 1.10 |
| 1 | 250 | 250 | 150 | 16766 | 8741 | 1.84 |
| 1/2 | 250 | 500 | 150 | 16389 | 8460 | 5.00 |
| 1/10 | 50 | 500 | 150 | 16682 | 8505 | 3.88 |
| 1/50 | 10 | 500 | 150 | 16284 | 7219 | 1.11 |
| 1/2 | 50 | 100 | 150 | 15279 | 7595 | 2.97 |
| 1/3 | 50 | 150 | 150 | 12960 | 6561 | 3.07 |
| 1/4 | 50 | 200 | 150 | 13033 | 6625 | 3.31 |
| 1/5 | 50 | 250 | 150 | 13444 | 6830 | 3.41 |
| 1/2 | 50 | 100 | 125 | 12988 | 6783 | 3.03 |
| 1/3 | 50 | 150 | 125 | 14931 | 7620 | 3.08 |
| 1/4 | 50 | 200 | 125 | 14073 | 7132 | 2.95 |
| 1/5 | 50 | 250 | 125 | 14470 | 7279 | 3.04 |
| 1/2 | 50 | 100 | 100 | 16085 | 8173 | 2.88 |
| 1/3 | 50 | 150 | 100 | 14553 | 7446 | 3.06 |
| 1/4 | 50 | 200 | 100 | 14208 | 7262 | 3.16 |
| 1/5 | 50 | 250 | 100 | 14847 | 7558 | 3.38 |
| 1/2 | 50 | 100 | 80 | 14590 | 7332 | 2.81 |
| 1/3 | 50 | 150 | 80 | 15282 | 7752 | 3.18 |
| 1/4 | 50 | 200 | 80 | 13093 | 6454 | 2.55 |
| 1/5 | 50 | 250 | 80 | 16817 | 8742 | 3.02 |
| 1/2 | 50 | 100 | 70 | 15977 | 8146 | 2.70 |
| 1/3 | 50 | 150 | 70 | 17009 | 8697 | 2.82 |
| 1/4 | 50 | 200 | 70 | 14384 | 7426 | 2.84 |
| 1/5 | 50 | 250 | 70 | 14825 | 7583 | 3.01 |
| 1/2 | 50 | 100 | 50 | 15920 | 8080 | 2.46 |
| 1/3 | 50 | 150 | 50 | 14623 | 7377 | 2.76 |
| 1/4 | 50 | 200 | 50 | 15694 | 8109 | 2.77 |
| 1/5 | 50 | 250 | 50 | 16704 | 8543 | 2.54 |
| 1/5 | 100 | 500 | 150 | 16349 | 8217 | 3.04 |
| 1/6 | 100 | 600 | 150 | 13598 | 6918 | 3.12 |
| 1/7 | 100 | 700 | 150 | 14969 | 7651 | 3.34 |
| 1/8 | 100 | 800 | 150 | 15688 | 8099 | 3.38 |
| 1/9 | 100 | 900 | 150 | 14916 | 7715 | 3.41 |
| 1/5 | 100 | 500 | 125 | 16056 | 8341 | 3.07 |
| 1/6 | 100 | 600 | 125 | 14535 | 7307 | 3.01 |
| 1/7 | 100 | 700 | 125 | 15850 | 8009 | 2.95 |
| 1/8 | 100 | 800 | 125 | 12592 | 6134 | 3.14 |
| 1/9 | 100 | 900 | 125 | 13710 | 6698 | 2.96 |
| 1/5 | 100 | 500 | 100 | 13355 | 6731 | 2.87 |
| 1/6 | 100 | 600 | 100 | 12180 | 6002 | 3.08 |
| 1/7 | 100 | 700 | 100 | 11448 | 5608 | 3.11 |
| 1/8 | 100 | 800 | 100 | 12930 | 6409 | 2.98 |
| 1/9 | 100 | 900 | 100 | 14766 | 7581 | 3.05 |
| 1 | 100 | 100 | 125 | 16810 | 8499 | 1.35 |
| 1 | 200 | 200 | 125 | 17137 | 4433 | 0.71 |
| 1 | 300 | 300 | 125 | 17090 | 0 | 0.00 |
| 1 | 400 | 400 | 125 | 15861 | 6 | - |
| 1 | 500 | 500 | 125 | 15301 | 0 | - |
| 1 | 600 | 600 | 125 | 12935 | 0 | - |
| 1 | 700 | 700 | 125 | 17229 | 0 | - |
| 1 | 800 | 800 | 125 | 17136 | 0 | - |
| 1 | 900 | 900 | 125 | 14990 | 0 | - |

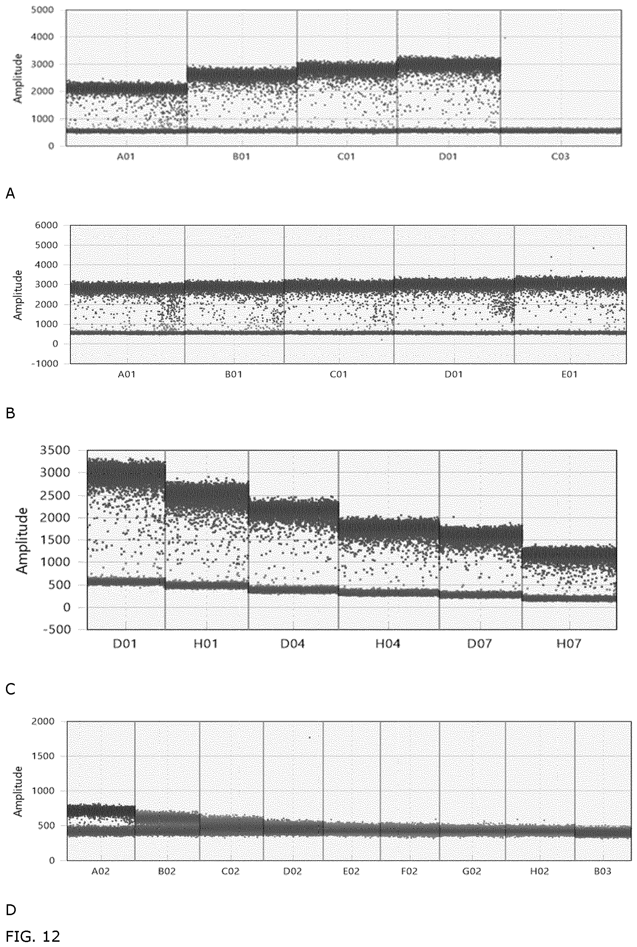

**Figure 12A** shows increasing resolution with increasing REG:MOD primer concentration while maintaining the probe concentration at 150 nM (left to right: 1:2, 1:3, 1:4, 1:5 MOD:REG, NTC). Primer ratios of 1:3, 1:4, and 1:5 result in optimal separability.

**Figure 12B** shows no further increase in resolution with higher REG:MOD primer ratios using the same probe concentration of 150 nM (left to right: 1:5, 1:6, 1:7, 1:8, 1:9 MOD:REG). These higher ratios are not favorable as low REG concentrations are preferable for avoiding non-specific amplification.

**Figure 12C** shows decreasing amplitude with decreasing probe concentration with constant MOD:REG primer ratio of 1:5 (from left to right: 150 nM, 125 nM, 100 nM, 80 nM, 70 nM, and 50 nM). Probe concentrations higher than the MOD primer concentration is recommended.

**Figure 12D** shows decrease in peak resolution with increasing primer concentration at a constant probe concentration of 125 nM (left to right: 1:1, 2:2, 3:3, 4:4, 5:5, 6:6, 7:7, 8:8, 9:9 MOD:REG, with 1:1 being 100:100 nM). The concentration of the modified primer should be lower than that of the regular primer (asymmetric PCR) to have good separability. Furthermore, probe concentrations are preferably higher than the modified primer concentration.

### Example 4: Cycling protocol with slow cooling

Slow cooling after final denaturation at the end of the PCR increases the separability of a digital PCR assay. **Figure 13** shows three assays with either slow or fast cooling (TOMM7, NOP10 and BRAF-WT) with their respective probes (PXL-RBO-001-P-FAM or PXL-RBO-001-B-HEX). Slow cooling was 0.5 °C/s and fast cooling 4 °C/s. **Figure 13A** shows TOMM7 and PXL-RBO-001-P-FAM with slow cooling (0.5 °C/s). **Figure 13B** shows TOMM7 and PXL-RBO-001-P-FAM with fast cooling (4 °C/s). **Figure 13C** shows NOP10 and BRAF-WT with probe PXL-RBO-001-B-HEX with slow cooling (0.5 °C/s). **Figure 13D** shows NOP10 and BRAF-WT with probe PXL-RBO-001-B-HEX with fast cooling (4 °C/s). The cycling was done on a T100 thermal cycler (Bio-Rad) and imaged on a QX200 (Bio-Rad).

### Example 5: dPCR and qPCR

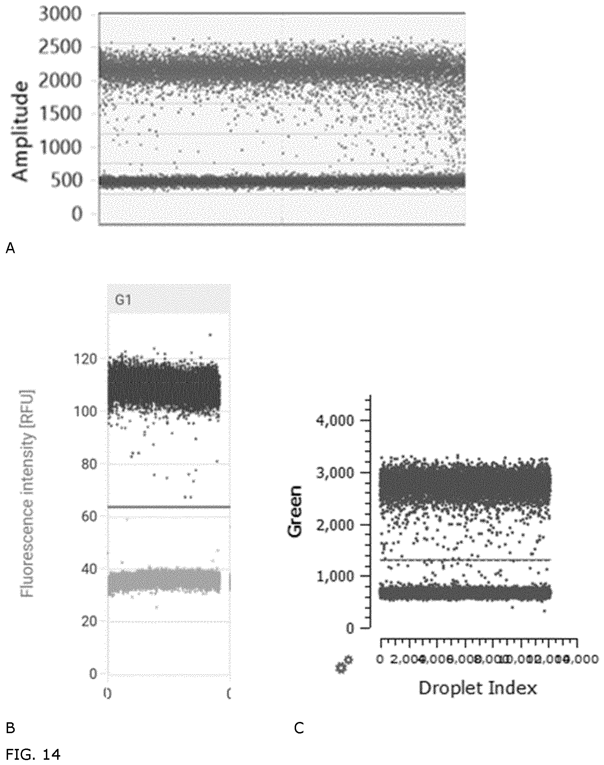

The probes of the current invention work on different instruments for both qPCR and dPCR as shown in the examples discussed above. Additional examples of different platforms are shown in Figure 14. **Figure 14A** shows results for probe PXL-RBO-001-G-ATTO590 on the QX600 (Bio-Rad). **Figure 14B** shows results for probe PXL-RBO-001-O-Cy5 on the QIAcuity (Qiagen). **Figure 14C** shows results for probe PXL-RBO-001-B-HEX on the Nio+ system (Stilla Technologies).

### Example 6: PCR detection systems according to an embodiment of the invention comprising one or more allele-specific modified primers

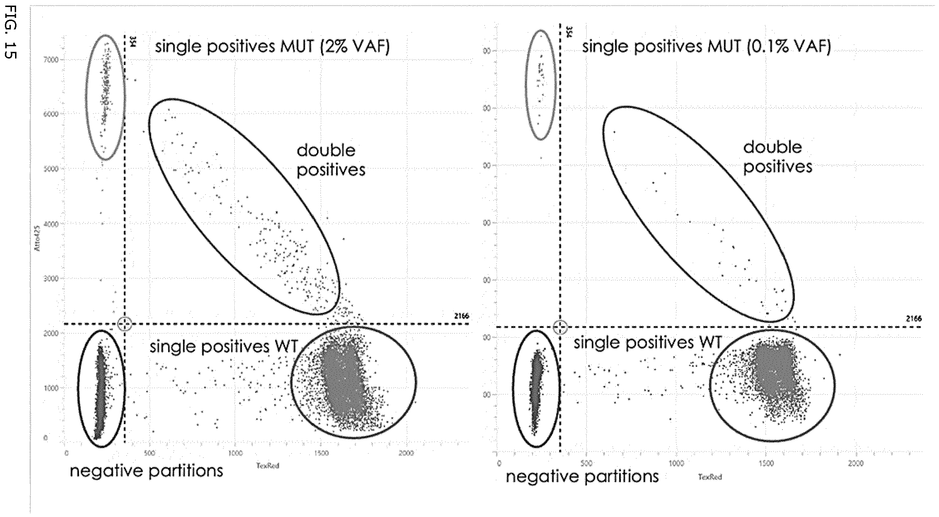

**Figure 15** shows a PCR assay to detect the BRAF V600E mutation according to an embodiment of the invention having an allele-specific primer that is modified for detection with a non-target specific probe through 5' end modification of the primer. A published BRAF V600E singleplex SYBR Green I primer (Lefever et al., Scientific Reports, 2019) was modified (see below how) to be compatible with universal probes and duplexing (simultaneous detection of mutant (MUT) and wild-type (WT) allele). Two samples are shown with 2 and 0.1% VAF detected with this assay, demonstrating the possibility to amplify and detect low abundant mutations selectively.

To further increase the selectivity, an artificial destabilizing mismatch is introduced in the 3' end of the primer. For adequate selectivity, it is crucial that the position or the nucleotide of the artificial mismatch in the WT and MUT allele-specific primer is different. When it is on the same position ánd has the same mismatch nucleotide, the effect of the destabilizing mismatch is neutralized after the first cycle. By differing the position (or the nucleotide), the number of mismatches increases to 3 (for a WT primer on the MUT template, and vice versa) after cycle 1, which greatly boosts the selectivity of the assay.

**Figure 1** shows a visual representation of an assay according to an embodiment of the invention. The primer pair exists of 2 target specific primers, of which one is modified at its 5' end with a 5'-universal tail sequence (number 1). The tail sequence has the same sequence as a dual-labeled hybridization probe. When the unmodified primer gets extended during PCR, a complementary sequence is created of the 5'-universal tail, to which the hairpin probe can bind, resulting in fluorescence (because the stem opens and fluorophore and quencher are separated). The DNA template is depicted in light grey with possible mismatch positions (4, 3 and 2) and with the targeted mutation at the 3' end (position 0). Primers are depicted in dark grey with the mutation specific primer on the top left with the universal probe modification (the 5' tail). In a duplex assay, WT (Wild Type) and MUT (mutant) primer differ with their 5' end probe modification and mismatch position or sequence. For example, WT primer can have a mismatch at position 4 with the WT template and MUT primer can have a mismatch at position 2 with the MUT template. This combination is written as 4/2 (see table 5). This results into nine different mismatch position based combinations. More allele-specific primers are possible (that will result in 3 nucleotides difference as of cycle 2) if also different nucleotides are considered as destabilizing nucleotides for the wild-type or mutant primer.

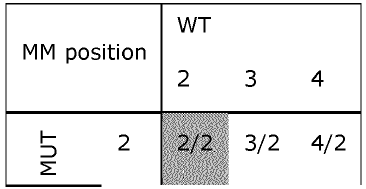

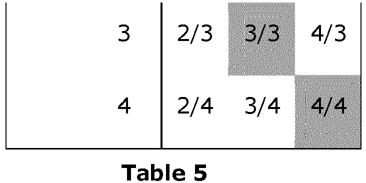

The above mentioned 9 position possibilities were tested in singleplex using qPCR with either WT assay and WT input (square), WT assay and MUT input (diamond), MUT assay and WT input (triangle) and MUT assay and MUT input (circle).

Assays were designed for a mutation in the AXIN1 (**Figure 16**) or PTPRD gene (**Figure 17**). Amplification plots are shown for each mismatch combination. Their respective ΔCq values are depicted in table 6 below. The higher the ΔCq value, the more selective an assay is for a specific input.

When the 2nd mismatch is made on the same position for the WT and MUT primer, very low specificity is observed. Varying this position greatly increases the specificity, demonstrating the absolute need to use different destabilizations in the mutant and wild-type primer.

| Figure | 16A | 16B | 16C |
|---|---|---|---|
| position in WT | 2 | 3 | 4 |
| position in MUT | 2 | 3 | 4 |
| ΔCq WT | 2.4 | 2.38 | 2.02 |
| ΔCq MUT | 0.7 | 1.0 | 1.0 |

| Figure | 16D | 16E | 16F |
|---|---|---|---|
| position in WT | 2 | 2 | 3 |
| position in MUT | 3 | 4 | 2 |
| ΔCq WT | 12.9 | 13.8 | 12.7 |
| ΔCq MUT | 21.8 | 21.6 | 22.2 |

| Figure | 16G | 16H | 16I |
|---|---|---|---|
| position in WT | 3 | 4 | 4 |
| position in MUT | 4 | 2 | 3 |
| ΔCq WT | 12.4 | 13.2 | 10.9 |
| ΔCq MUT | 6.6 | 21.7 | 21.3 |

| Figure | 17A | 17B | 17C |
|---|---|---|---|
| position in WT | 2 | 3 | 4 |
| position in MUT | 2 | 3 | 4 |
| ΔCq WT | 8.22 | 10.14 | 10.6 |
| ΔCq MUT | 0.7 | 2.8 | 1.4 |

| Figure | 17D | 17E | 17F |
|---|---|---|---|
| position in WT | 2 | 2 | 3 |
| position in MUT | 3 | 4 | 2 |
| ΔCq WT | 24.0 | 12.9 | 5.8 |
| ΔCq MUT | 8.4 | 19.7 | 21.8 |

**Table 6**

| Figure | 17G | 17H | 17I |
|---|---|---|---|
| position in WT | 3 | 4 | 4 |
| position in MUT | 4 | 2 | 3 |
| ΔCq WT | 8.4 | 6.2 | 23.6 |
| ΔCq MUT | 9.7 | 2.2 | 8.8 |

### Example 7: Optimization of thermocycling conditions

Three cycle protocols were tested to determine the optimal duration of the denaturation and elongations step (see table 7 below).

**Table 7**

| PCR step | temperature (°C) | cycle protocol 1 | cycle protocol 2 | cycle protocol 3 |
|---|---|---|---|---|
| 1 | 95 | 10 min | 10 min | 10 min |
| 2 | 94 | 1 sec | 5 sec | 5 sec |
| 3 | 60 | 1 sec | 10 sec | 30 sec |
| 4 | 98 | 10 min | 10 min | 10 min |
| 5 | 12 | ∞ | ∞ | ∞ |

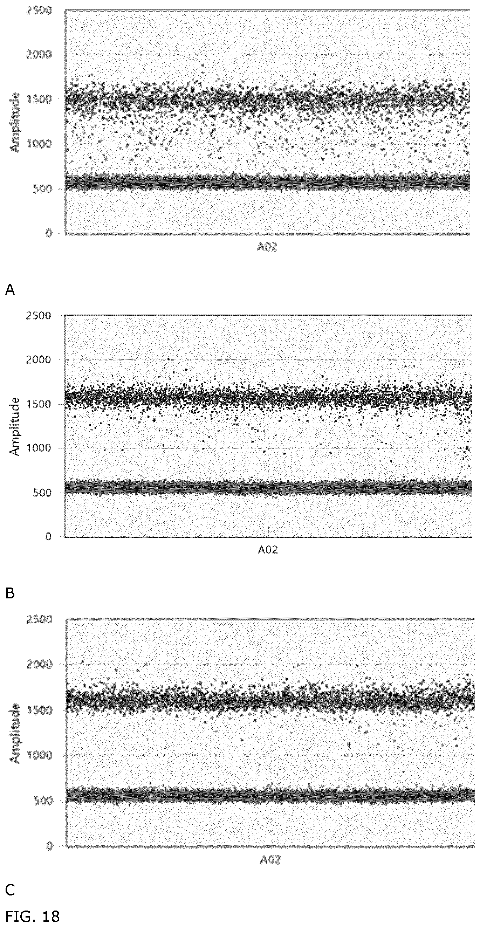

The results of cycle protocol 1 is depicted in **Figure 18A****,** cycle protocol 2 is depicted in **Figure 18B****,** cycle protocol 1 is depicted in **Figure 18C****.** Longer denaturation (5 versus 1 second) and longer elongation (30 versus 10 or 1 second) result in better separability.

The present invention is in no way limited to the embodiments described in the examples and/or shown in the figures. On the contrary, methods according to the present invention may be realized in many different ways without departing from the scope of the invention.

## Claims

1. A PCR detection system comprising a reaction mixture for analyzing, quantitating or detecting a target nucleic acid, wherein the reaction mixture comprises:
a. a first primer, wherein said first primer comprises a region configured to selectively hybridize to said target nucleic acid under amplification conditions and a first 5'-universal tail which is not complementary to the target nucleic acid sequence;
b. a first universal detection probe, wherein said first universal detection probe comprises a first fluorophore,
**characterized in that** the first 5'-universal tail of said first primer comprises a nucleotide sequence, the complement of which allows selective hybridization to a nucleotide sequence comprised in said first universal detection probe.

2. A PCR detection system according to claim 1, wherein said 5'-universal tail of said first primer comprises a nucleotide sequence having a sequence identity of at least 90% to a nucleotide sequence comprised in said first universal detection probe, wherein said nucleotide sequence showing sequence identity comprises at least 8 nucleotides.

3. A PCR detection system according to any of the previous claims, wherein said first primer is a first allele-specific primer, which is specific for a first allele of the target nucleic acid.

4. A PCR detection system according to any of the previous claims, further comprising:
a. a second primer, wherein said second primer is a second allele-specific primer, which is specific for a second allele of the target nucleic acid and wherein said second primer comprises a region configured to selectively hybridize to said target nucleic acid under amplification conditions and a second 5'-universal tail which is not complementary to the target nucleic acid sequence;
b. a second universal detection probe, wherein said second universal detection probe comprises a second fluorophore, wherein said second 5'-universal tail of said second primer comprises a nucleotide sequence, the complement of which allows selective hybridization to a nucleotide sequence comprised in said second universal detection probe and wherein the first and second fluorophore are different.

5. A PCR detection system according to any of the previous claims 3-4, wherein said allele-specific primer(s) have a 3'-terminal interrogating nucleotide.

6. A PCR detection system according to any of the previous claims 3-5, wherein said first allele-specific primer and/or said second allele-specific primer further include a deliberately introduced nucleotide near its 3' end in the region configured to selectively hybridize to said target nucleic acid, wherein said introduced nucleotide is mismatched to the target nucleic acid to destabilize the primer and increase its allele discrimination.

7. A PCR detection system according to any of the previous claims 4-6, wherein said first allele-specific primer and said second allele-specific primer each include a deliberately introduced nucleotide near its 3' end in the respective regions configured to selectively hybridize to said target nucleic acid, wherein each of said introduced nucleotides are mismatched to the target nucleic acid and wherein said mismatched nucleotide in said first allele-specific primer is different from said mismatched nucleotide in said second allele-specific primer.

8. A PCR detection system according to any of the previous claims, wherein said first and/or second universal detection probe comprises a molecular beacon probe comprising both a fluorophore and a quencher moiety.

9. A PCR method for quantifying a target nucleic acid molecule by means of a PCR detection system of any of the previous claims 1-8, the method comprising: providing a reaction mixture comprising one or more target nucleic acid molecules and a primer, wherein said primer comprises a region configured to selectively hybridize to said target nucleic acid under amplification conditions and a 5'-universal tail which is not complementary to the target nucleic acid sequence, a universal detection probe, wherein said universal detection probe comprises a fluorophore, wherein the 5'-universal tail of said primer comprises a nucleotide sequence, the complement of which allows selective hybridization to a nucleotide sequence comprised in said universal detection probe; amplifying the target nucleic acid; measuring the fluorophore and thereby quantifying the target nucleic acid molecule.

10. A PCR method for determining the genotype of a target nucleic acid molecule by means of a PCR detection system of any of the previous claims 4-8, the method comprising: providing a reaction mixture comprising one or more target nucleic acid molecules and a first allele-specific primer which is specific for a first allele, said first allele-specific primer comprising a first 5'-universal tail which comprises a nucleotide sequence, the complement of which allows selective hybridization to a nucleotide sequence comprised in a first universal detection probe, wherein the first universal detection probe comprises a first fluorophore; and a second allele-specific primer which is specific for a second allele, said second allele-specific primer comprising a second 5'-universal tail which comprises a nucleotide sequence the complement of which allows selective hybridization to a nucleotide sequence comprised in a second universal detection probe, wherein the second universal probe comprises a second fluorophore; wherein the first and second fluorophore are different; amplifying the target nucleic acids; measuring the intensities of the first and the second fluorophore; and analyzing nucleic acid genotypes based on the intensities of the first and second fluorophores.

11. Method according to claim 10, wherein said first allele-specific primer and said second allele-specific primer each include a deliberately introduced nucleotide near its 3' end that is mismatched to the target nucleic acid to destabilize the primer and increase its allele discrimination and wherein said mismatched nucleotide is different in said first allele-specific primer and said second allele-specific primer.

12. A universal detection probe comprising a fluorophore, wherein at least part of said universal detection probe forms a loop, **characterized in that** the nucleotide sequence of said loop comprises:
- a length of between 8 and 24 nucleotides;
- a GC content between 55 and 70%;
- a melting temperature between 60°C and 70 °C;
and wherein said nucleotide sequence does not:
- comprise more than three consecutive identical nucleotides;
- comprise a deoxycytosine (C) or deoxyguanosine (G) at the 5' end of the loop;
- form a secondary structure;
- show complementarity to the human genome;
- or show complementarity to itself.

13. Universal detection probe according to claim 12, wherein said loop comprises 18 nucleotides.

14. Use of the universal detection probe according to any of the previous claims 12-13 in a PCR detection system or a PCR method according to any of the previous claims 1-11, wherein said universal detection probe is used in combination with a universal primer comprising a 5'-universal tail, wherein the nucleotide sequence of said loop allows selective hybridization to the complement of a nucleotide sequence comprised in said 5'-universal tail of said primer.

15. Use of the universal detection probe according to any of the previous claim 12-13, wherein said loop comprises a nucleotide sequence having a sequence identity of at least 90% to a nucleotide sequence comprised in said 5'-universal tail of said primer, wherein said nucleotide sequence showing sequence identity comprises at least 8 nucleotides.
